(19) Europäisches Patentamt
European Patent Office
Office européen des brevets

(11) **EP 4 560 305 A1**

(12) **EUROPÄISCHE PATENTANMELDUNG**

(43) Veröffentlichungstag:
**28.05.2025   Patentblatt 2025/22**

(21) Anmeldenummer: 23211581.6

(22) Anmeldetag: **22.11.2023**

(51) Internationale Patentklassifikation (IPC):
**G01N 27/406** (2006.01)     **G01N 27/407** (2006.01)

(52) Gemeinsame Patentklassifikation (CPC):
**G01N 27/4062**; G01N 27/407; G01N 33/0009

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Siemens Aktiengesellschaft
80333 München (DE)**

(72) Erfinder:
• **Baader, Mathias
76437 Rastatt (DE)**

• **Bindig, Heike
76275 Ettlingen (DE)**
• **Sperrfechter, Frank
76470 Ötigheim (DE)**
• **Weida, Simon
76831 Göcklingen (DE)**

(74) Vertreter: **Siemens Patent Attorneys
Postfach 22 16 34
80506 München (DE)**

Bemerkungen:
Geänderte Patentansprüche gemäss Regel 137(2) EPÜ.

(54) **ANORDNUNG MIT ELEKTRISCH LEITFÄHIGEN ZULEITUNGEN, EINER UMMANTELUNG UND EINEM STABFÖRMIGEN ISOLIERKÖRPER**

(57)     Anordnung mit Zuleitungen. Anordnung umfassend mindestens eine erste elektrisch leitfähige Zuleitung (1a, 1b, 1c), eine Ummantelung (2) und einen stabförmigen Isolierkörper (3); wobei die Ummantelung (2) ein erstes Ende (4a) und ein zweites Ende (4b) aufweist und das erste Ende (4a) verschieden ist von dem zweiten Ende (4b) und das erste Ende (4a) dem zweiten Ende (4b) gegenüberliegt und die Ummantelung (2) eine erste Öffnung an dem ersten Ende (4a) und eine zweite Öffnung an dem zweiten Ende (4b) aufweist; wobei die mindestens eine erste Zuleitung (1a, 1b, 1c) von dem ersten Ende (4a) zum zweiten Ende (4b) durch die Anordnung hindurch verläuft und eine Achse definiert; wobei der stabförmige Isolierkörper (3) mindestens einen Durchgang durch den stabförmigen Isolierkörper (3) umfasst und ein erster Abschnitt der mindestens einen Zuleitung (1a, 1b, 1c) in dem mindestens einen Durchgang durch den stabförmigen Isolierkörper (3) verläuft.

FIG 1

**Beschreibung**

Hintergrund

Die vorliegende Offenbarung bezieht sich auf eine Anordnung mit Zuleitungen für Sensorelemente. Insbesondere bezieht sich die vorliegende Offenbarung auf eine solche Anordnung mit Zuleitungen zum Einsatz in einer Verbrennungsvorrichtung. Das Sensorelement kann beispielsweise eine Gaskonzentration, insbesondere eine Sauerstoffkonzentration, aufzeichnen.

Industrielle Prozesse verwenden Energiewandlung durch Verbrennung, um Dampf und/oder Wärme für einen industriellen Prozess zu erzeugen. Dazu brennt im Feuerraum einer Verbrennungsvorrichtung im Betrieb eine Flamme eines Wärmeerzeugers. Der Wärmeerzeuger tauscht die Wärmeenergie der heissen Brenngase in ein anderes Fluid wie beispielsweise Wasser. Mit dem warmen Wasser wird beispielsweise eine Warmwasserheizungsanlage betrieben und/oder Trinkwasser erwärmt. Gemäss einer anderen Ausführungsform kann mit der Wärmeenergie der heissen Brennstoffe und/oder Brenngase ein Gut beispielsweise in einem industriellen Prozess erhitzt werden. Gemäss einer weiteren Ausführungsform ist der Wärmeerzeuger Teil einer Anlage mit Kraft-Wärme-Kopplung, beispielsweise ein Motor einer solchen Anlage. Ferner kann der Wärmeerzeuger der Erhitzung von Wasser in einer Anlage zur Gewinnung von Lithium und/oder Lithiumkarbonat dienen. Die Abgase werden aus dem Feuerraum beispielsweise über einen Abgaskamin und/oder einen Rauchgaskamin und/oder einen Schornstein abgeführt. In dem Abgaskamin und/oder Rauchgaskamin und/oder Schornstein ist dann ein Sensorelement angeordnet, welches beispielsweise eine Sauerstoffkonzentration aufzeichnen kann.

Einige solche Prozesse beinhalten den Betrieb eines Ofens oder Kessels. Während die Verbrennung eine kostengünstige Energiewandlung darstellt, wird häufig versucht, die Verbrennungseffizienz innerhalb eines Prozesses zu maximieren. Die Maximierung der Verbrennungseffizienz ist unter anderem eine Folge der resultierenden Abgase und/oder Rauchgase, die das System verlassen. Jene Abgase und/oder Rauchgase unterliegen bisweilen Vorschriften bezüglich der Emissionen schädlicher Gase. Mithin besteht ein Ziel der Optimierung darin, die Verbrennungseffizienz bestehender Öfen und/oder Kessel zu maximieren. Damit einher geht eine Reduktion der Produktion von Treibhausgasen und anderen schädlichen Nebenprodukten.

Ein weiteres Ziel ist die Optimierung für verschiedene Brennstoffe und/oder Brenngase. Insbesondere geht es dabei um Brennstoffe und/oder um Brenngase, welche Wasserstoffgase umfassen. Vorteilhaft geht es um Brennstoffe und/oder um Brenngase, welche bei 293 Kelvin mehr als zwanzig Volumenprozent Wassersstoff umfassen. In einigen Fällen liegt bei 293 Kelvin der Anteil an Wasserstoffgas bei fünfzig Volumenprozent oder gar bei siebzig Volumenprozent.

Die Verbrennungseffizienz kann optimiert werden, indem auf den Sauerstoffgehalt in den aus einem Verbrennungsprozess stammenden Abgasen und/oder Rauchgasen geregelt wird. Damit wird die Oxidation der Verbrennungsnebenprodukte weitgehend sicherstellt.

In-situ- oder In-Prozess-Analysegeräte können beim Überwachen und/oder Optimieren und/oder Steuern eines laufenden Verbrennungsprozesses eingesetzt werden. Gängige Analysegeräte umfassen eine Sensoreinheit. Die Sensoreinheit wird auf hohe Temperaturen erhitzt. Sie arbeitet direkt in der Verbrennungszone oder nahe der Verbrennungszone des Ofens oder Kessels.

Bekannte Analysegeräte verwenden typischerweise einen auf Zirconiumdioxid basierenden Sauerstoffsensor. Der Sauerstoffsensor ist an einem Ende einer Sonde angeordnet, die in einen Rauchgasstrom eingeführt wird. Wenn das Abgas und/oder Rauchgas in das Analysegerät strömt, diffundiert es durch einen Filter oder Diffusor in die Nähe des auf Zirconiumdioxid basierenden Sauerstoffsensors. Es gibt keine Pumpen und/oder andere strömungsinduzierende Vorrichtungen, die verwendet werden, um den Probenfluss in das Analysegerät zu leiten. Stattdessen dringt das Gas passiv durch den Diffusor. Der Sensor liefert ein elektrisches Signal, das eine im Abgas und/oder Rauchgas vorhandene Sauerstoffmenge angibt.

Der auf Zirconiumdioxid basierende Sauerstoffsensor bietet eine potentiometrische Indikation. Die potentiometrische Indikation gilt als zuverlässige Sauerstoffmessung in Verbrennungsumgebungen. Sie ermöglicht eine effiziente und/oder sichere Prozesssteuerung. Typischerweise wird eine einzelne Sonde in den Prozess beispielsweise in den Abgaskamin und/oder in den Rauchgaskamin und/oder in den Schornstein eingeführt. Eine prozentuale Sauerstoffmessung wird verwendet, um die Verbrennungseffizienz in kleinen Kesseln und/oder Öfen zu optimieren. Bei grossen Anlagen trifft der Betreiber vielfach auf eine Abgasschichtung und/oder Rauchgasschichtung. Die Abgasschichtung und/oder Rauchgasschichtung umfasst eine Vielzahl an Schichten mit jeweils unterschiedlicher Sauerstoffkonzentration.

Um Schichtungsinformationen zu erhalten, können Betreiber für einen effizienten und sicheren Betrieb mehrere Sonden in den Abgaskamin und/oder in den Rauchgaskamin und/oder in den Schornstein einbauen. Es können in einzelnen Fällen bis zu sechzehn solche Sonden eingebaut werden.

Es werden dabei nicht nur an die Sensoreinheit hohe Anforderungen in Bezug auf Temperaturbeständigkeit und Widerstand gegenüber chemischer Zersetzung gestellt. Gleiche oder ähnliche Anforderungen müssen die Zuleitungen zur Sensoreinheit erfüllen. Insbesondere müssen Zuleitungen, welche die Sensoreinheit elektrisch kontaktieren, temperaturbeständig und widerstandsfähig gegenüber chemischer Zersetzung sein.

Ein publiziertes Gebrauchsmuster CN2685875Y aus China wurde am 17. Juli 2003 eingereicht. Das Gebrauchsmuster CN2685875Y wurde am 16. März 2005 publiziert. Die Schrift CN2685875Y befasst sich mit einem integralen Rauchanalysegerät auf Basis von Zirconiumdioxid. Die Schrift CN2685875Y offenbart ein Analysegerät mit einer Messspitze. An einem ersten Ende der Messspitze sind ein Sensor auf Basis von Zirconiumdioxid, eine Heizvorrichtung und ein Temperatursensor angebracht. An einem zweiten Ende der Messspitze befinden sich zwei Gasanschlüsse sowie ein Gehäuse. Aus dem Gehäuse ragt ein Auslass. CN2685875Y offenbart Massnahmen zum Schutz der Zuleitungen zu dem Sensor auf Basis von Zirconiumdioxid und zum Schutz der Zuleitungen zu dem Temperatursensor. Zu den Massnahmen gehören ein Messrohr, welches an einem Flansch befestigt ist, und einen Regenschutz.

Eine internationale Patentanmeldung WO2022/064271A1 wurde eingereicht am 11. Dezember 2020 durch ROSEMOUNT INC. Die Anmeldung wurde veröffentlicht am 31. März 2022. WO2022/064271A1 nimmt eine Priorität vom 24. September 2020 in Anspruch. Die Anmeldung WO2022/064271A1 befasst sich mit einem In-situ-Analysegerät mit Mittelwertbildung. WO2022/064271A1 offenbart ein Analysegerät mit einer Messspitze. Die Messspitze weist ein erstes und ein zweites Ende auf. Zwischen dem ersten und dem zweiten Ende der Messspitze befinden sich mehrere Öffnungen. Nahe dem zweiten Ende der Messspitze befinden sich eine Sensoreinheit und ein Flansch zur Befestigung des Analysegerätes. Gemäss WO2022/064271A1 benötigt jedes Sensorelement Zuleitungen und/oder Signalleitungen.

Eine Patentanmeldung DE102012211039A1 wurde eingereicht am 27. Juni 2012 durch die Robert Bosch GmbH, 70469, Stuttgart. Die Anmeldung wurde veröffentlicht am 2. Januar 2014. DE102012211039A1 behandelt einen Gassensor für Russ. Dabei ist eine Sensoreinheit in einem Schutzrohr angeordnet. Ferner ist ein Befestigungsstutzen mit einem gegenüber einem Durchmesser eines Gehäuses reduziertem Durchmesser vorgesehen.

Das Patent US6015533A ist erteilt am 18. Januar 2000 an Motorola Inc (Schaumburg, IL). Es behandelt ein Sensorgehäuse für einen kalorimetrischen Sensor. Anmeldetag von US6015533A ist der 14. November 1997. US6015533A offenbart einen Sensor, der über mehrere elektrische Kabel angeschlossen ist. Ein Kabelgeschirr stützt die elektrischen Kabel innerhalb eines Rohres. Dabei begrenzt eine Wand den Abgasraum. Die Kabel und das Kabelgeschirr sind ausserhalb der Wand so angeordnet, dass die Kabel und das Kabelgeschirr und dessen Umhüllung nicht dem Abgasstrom ausgesetzt sind.

Eine Patentanmeldung US2010/050738A1 wurde eingereicht am 26. August 2008 Die Anmeldung wurde veröffentlicht am 4. März 2010. US2010/050738A1 behandelt eine Sensoranordnung mit einem thermisch isolierenden Gehäuse. Die Anordnung ist aufgeteilt in einen ersten und einen zweiten zylindrischen Abschnitt. Zwischen dem ersten und dem zweiten zylindrischen Abschnitt liegt ein Flansch. Der erste zylindrische Abschnitt umfasst eine Einlass- und eine Auslassöffnung. Der zweite zylindrische Abschnitt umgibt eine Mehrzahl von Kabeln. Ähnlich dem Kabelgeschirr aus US6015533A sind die Kabel aus US2010/050738A1 anhand einer Kabeltülle geführt. Die Kabeltülle umfasst dabei Durchgänge für die einzelnen Kabel.

Eine europäische Patentanmeldung EP4236640A1 wurde eingereicht am 23. Februar 2022 durch die SIEMENS AG. Die Anmeldung wurde veröffentlicht am 30. August 2023. EP4236640A1 behandelt eine Halterung für eine Leiterplatte. Die Halterung aus EP4236640A1 umfasst mindestens ein rohrförmiges Befestigungselement. Innerhalb des mindestens einen rohrförmigen Befestigungselementes ist mindestens ein elektrischer Leiter angeordnet. Das mindestens eine rohrförmige Befestigungselement ist durch eine erste Öffnung der Halterung geführt.

Eine Patentanmeldung CN115791931A wurde eingereicht am 29. November 2022. Die Anmeldung wurde veröffentlicht am 14. März 2023. CN115791931A offenbart eine Packstruktur für einen Sauerstoffsensor im Hinblick auf die industrielle Automatisierung eines Verbrennungsprozesses. Insbesondere offenbart CN115791931A einen Keramikring innerhalb eines Rohres. Eine Isolierstück aus Keramik weist gemäss CN115791931A eine Vielzahl von Durchführungen auf. In diesen Durchführungen verlaufen Platindrähte.

Ziel der vorliegenden Offenbarung ist es, eine Anordnung mit einer oder mehreren Zuleitungen bereitzustellen, welche eine Gasanalyse an einer Verbrennungsvorrichtung ermöglicht. Dazu ist die Anordnung so auszulegen, dass sie den thermischen wie auch mechanischen und chemischen Belastungen im Betrieb standhält.

Zusammenfassung

Es wird eine Anordnung umfassend eine oder mehrere Zuleitungen bereitgestellt. Die eine oder die mehreren Zuleitungen verlaufen durch einen stabförmigen Isolierköper. Die eine oder die mehreren Zuleitungen verlaufen vorzugsweise parallel zueinander durch einen stabförmigen Isolierköper. Der stabförmige Isolierkörper umfasst dazu einen oder mehrere Durchgänge für die eine oder für die mehreren Zuleitungen. Anhand des einen oder der mehreren Durchgänge durch den stabförmigen Isolierkörper wird die eine oder werden die mehreren Zuleitungen geführt. Zudem wird oder werden anhand des einen oder der mehreren Durchgänge durch den stabförmigen Isolierkörper die eine oder die mehreren Zuleitungen fixiert.

Die eine oder die mehreren Zuleitungen können insbesondere so durch den stabförmigen Isolierkörper geführt werden, dass die Durchleitung gasdicht wird. Besonders bevorzugt wird die Anordnung gasdicht gegenüber Verbrennungsgasen aus einer Verbrennungsvorrichtung. Das heisst, dass der eine oder die mehreren Durchgänge die eine oder die mehreren Zuleitungen derart umschliessen, dass ein Durchtritt von Gasen wie beispielsweise Verbrennungsgasen behindert wird.

Die eine oder die mehreren Zuleitungen ist oder sind elektrisch leitfähig. Die eine oder die mehreren Zuleitungen ist oder sind aus einem Material mit hoher Temperaturbeständigkeit gefertigt. Dadurch eignet sich die Anordnung mit ihrer Zuleitung oder ihren Zuleitungen für den Einsatz in einer Verbrennungsvorrichtung.

Der stabförmige Isolierkörper ist aus einem elektrisch isolierenden und temperaturbeständigen Material gefertigt. Vorzugsweise weist der stabförmige Isolierkörper eine hohe Beständigkeit gegenüber chemischer Zersetzung durch Verbrennungsgase auf. Beispielsweise kann der stabförmige Isolierkörper aus einem keramischen Material gefertigt sein.

Eine Ummantelung umgibt den stabförmigen Isolierkörper und die eine oder die mehreren Zuleitungen. Die Ummantelung muss ebenfalls temperaturbeständig und beständig gegenüber chemischer Zersetzung sein. Beispielsweise kann die Ummantelung aus korrosionsbeständigem Stahl gefertigt sein. Zudem ist auf eine Kompatibilität des Materials der Ummantelung mit dem Material einer Aussenwand einer Verbrennungsvorrichtung zu achten. Solches ist wichtig, wenn die Ummantelung durch die Wand einer Verbrennungsvorrichtung geführt wird.

Zwischen der Ummantelung und dem stabförmigen Isolierkörper befindet sich ein Spalt mit einer von Null verschiedenen Dicke. Der Spalt kann beispielsweise mit Luft und/oder mit Verbrennungsgasen, allgemein mit einem Gas, gefüllt sein. Der Spalt verbessert die thermische Isolierung des stabförmigen Isolierkörpers und der Zuleitungen von der Ummantelung.

Abstandshalter beabstanden den stabförmigen Isolierkörper von der Ummantelung. Die Abstandshalter können einen Einfasskörper, insbesondere einen Vergusskörper, umfassen. Der Einfasskörper, insbesondere der Vergusskörper, ist nahe einem Ende der Ummantelung angeordnet und fixiert den stabförmigen Isolierkörper. Der Einfasskörper, insbesondere der Vergusskörper, kann zudem die Anordnung in Richtung jenes Endes gasdicht verschliessen.

In dem Einfasskörper, insbesondere in dem Vergusskörper, sind eine oder mehrere Durchgänge für die eine oder die mehreren Zuleitungen vorhanden.

Zu den Abstandshaltern können zudem einer oder mehrere Ringe gehören, welche den stabförmigen Isolierkörper von der Ummantelung beabstanden.

Die Anordnung eignet sich zum Einbau in eine Verbrennungsvorrichtung. Die eine Zuleitung oder die mehreren Zuleitungen eignet oder eignen sich zur galvanischen Verbindung mit einer Sensoreinheit innerhalb der Verbrennungsvorrichtung. Dazu wird die eine Zuleitung oder werden die mehreren Zuleitungen an ihrem Ende oder an ihren Enden galvanisch mit einer Sensoreinheit verbunden. Eine Sensoreinheit im Inneren der Verbrennungsvorrichtung kann so elektrisch kontaktiert werden.

Kurze Beschreibung der Zeichnungen

Verschiedene Merkmale werden dem Fachmann aus der folgenden detaillierten Beschreibung der offenbarten nicht einschränkenden Ausführungsformen ersichtlich. Die Zeichnungen, die der detaillierten Beschreibung beiliegen, können kurz wie folgt beschrieben werden:

FIG 1 zeigt schematisch eine Zuleitungsanordnung.

FIG 2 zeigt schematisch die Zuleitungsanordnung als Durchführung durch eine Wand.

Detaillierte Beschreibung

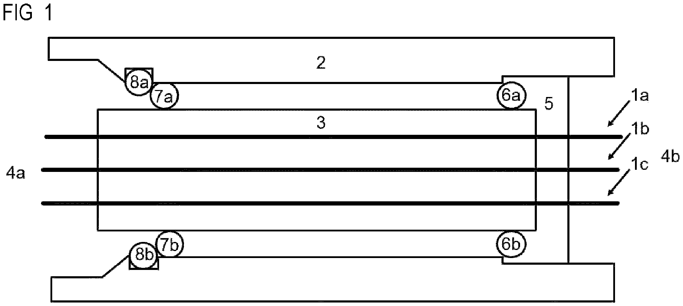

In FIG 1 ist eine Anordnung mit einer oder mehreren Zuleitungen 1a, 1b, 1c gemäss der vorliegenden Offenbarung veranschaulicht. Die Anordnung umfasst eine Ummantelung 2. Die Ummantelung 2 kann beispielsweise eine rohrförmige Ummantelung umfassen. Die Ummantelung 2 kann insbesondere eine rohrförmige Ummantelung sein. In einer speziellen Ausführungsform ist die Ummantelung 2 zylindersymmetrisch um eine Achse, welche parallel zu den Zuleitungen 1a, 1b, 1c verläuft.

Die Ummantelung 2 ist vorzugsweise aus Stahl gefertigt. Besonders bevorzugt ist die Ummantelung 2 aus korrosionsbeständigem Stahl gefertigt. Eine Ummantelung 2 aus korrosionsbeständigem Stahl verschafft Widerstandskraft gegen chemische Degradation in einem Abgaskanal.

Ferner kann die Ummantelung 2 aus ferritischem Stahl gefertigt sein. Darüber hinaus kann die Ummantelung 2 aus austenitischem Stahl gefertigt sein.

Mindestens eine der Zuleitungen 1a, 1b, 1c ist vorzugsweise hochtemperaturbeständig. Sie widersteht damit den Temperaturen in einem Abgaskanal einer Verbrennungsvorrichtung wie beispielsweise einem Gasbrenner. In einer hochtemperaturbeständigen Ausführungsform umfasst mindestens eine der Zuleitungen 1a, 1b, 1c Nickeldraht. In einer besonderen Ausführungsform besteht mindestens eine der Zuleitungen 1a, 1b, 1c aus Nickeldraht. Beispielsweise kann mindestens eine der Zuleitungen 1a, 1b, 1c aus einer Legierung bestehen, welche zu mehr als siebzig oder zu mehr als fünfundsiebzig Massenprozent aus Nickel besteht.

Idealerweise sind sämtliche Zuleitungen 1a, 1b, 1c hochtemperaturbeständig. Sie widerstehen damit den Temperaturen in einem Abgaskanal einer Verbrennungsvorrichtung wie beispielsweise einem Gasbrenner. In einer hochtemperaturbeständigen Ausführungsform umfassen sämtliche Zuleitungen 1a, 1b, 1c Nickeldraht. In einer besonderen Ausführungsform bestehen sämtliche Zuleitungen 1a, 1b, 1c aus Nickeldraht. Beispielsweise können sämtliche Zuleitungen 1a, 1b, 1c aus einer Legierung bestehen, welche zu mehr als siebzig oder zu mehr als fünfundsiebzig Massenprozent aus Nickel besteht.

Die Zuleitungen 1a, 1b, 1c haben jeweils einen Durchmesser, der kleiner als der geringste Innendurchmesser der Ummantelung 2 ist. Beispielsweise können die Zuleitungen Durchmesser von 0.5 Millimetern oder einem Millimeter aufweisen. Es sind auch Durchmesser möglich, welche grösser als ein Millimeter sind.

In einer Ausführungsform haben alle Zuleitungen 1a, 1b, 1c die gleichen Durchmesser. Gleiche Durchmesser verringern die Anzahl an Varianten der Anordnung. Mit der Anzahl an Varianten verringert sich auch das Risiko, dass eine der Varianten im Laufe ihres Betriebes ausfällt.

Innen in der Ummantelung 2 ist ein stabförmiger Isolierkörper 3 angeordnet. Der stabförmige Isolierkörper 3 ist vorzugsweise ein stabförmiger, elektrischer Isolierkörper und/oder eine stabförmige, elektrische Isolierung. Der stabförmige Isolierkörper 3 weist vorzugsweise einen spezifischen elektrischen Widerstand ρ von mindestens einem Megaohm · Zentimeter bei Temperaturen von 873 Kelvin auf:

$$\rho > 1\ M\Omega \cdot \mathrm{cm\ bei\ 873\ Kelvin}$$

Besonders bevorzugt ist der spezifische elektrische Widerstand ρ bei Temperaturen von 873 Kelvin grösser als fünf Megaohm · Zentimeter:

$$\rho > 5\ M\Omega \cdot \mathrm{cm\ bei\ 873\ Kelvin}$$

Weiterhin bevorzugt ist der spezifische elektrische Widerstand ρ bei Temperaturen von 873 Kelvin grösser als zehn Megaohm · Zentimeter:

$$\rho > 10\ M\Omega \cdot \mathrm{cm\ bei\ 873\ Kelvin}$$

Hohe spezifische Widerstände ermöglichen eine ausreichende elektrische Isolierung zwischen den Zuleitungen 1a, 1b, 1c. Jedwedes Signal, das aus einem an die Zuleitungen 1a, 1b, 1c angeschlossenen Sensorelement gewonnen wird, wird mithin wenig verfälscht.

Der stabförmige Isolierkörper 3 umfasst in einer Ausführungsform Keramik, insbesondere Aluminiumoxidkeramik. Der stabförmige Isolierkörper 3 besteht in einer speziellen Ausführungsform aus Keramik, insbesondere aus Aluminiumoxidkeramik. Vorzugsweise weist die Aluminiumoxidkeramik eine Reinheit von mehr als 92 Prozent auf. Besonders bevorzugt weist die Aluminiumoxidkeramik eine Reinheit von mehr als 96 Prozent auf. Idealerweise weist die Aluminiumoxidkeramik eine Reinheit von mehr als 99 Prozent auf. Eine verbesserte Reinheit der Keramik führt zu einem vorhersagbareren Verhalten in Bezug auf elektrische Isolierung und mechanische Festigkeit.

In einer anderen Ausführungsform umfasst der stabförmige Isolierkörper 3 einen keramischen Werkstoff auf der Basis von Magnesiumsilikat. Ferner kann der stabförmige Isolierkörper 3 aus einem keramischen Werkstoff auf der Basis von Magnesiumsilikat bestehen. In einer weiteren Ausführungsform umfasst der stabförmige Isolierkörper 3 Porzellan. Ferner kann der stabförmige Isolierkörper 3 aus Porzellan bestehen. In wiederum einer weiteren Ausführungsform umfasst der stabförmige Isolierkörper 3 Porzellan. Ferner kann der stabförmige Isolierkörper 3 aus Porzellan bestehen.

Der stabförmige Isolierkörper 3 weist mindestens einen Durchgang für eine der Zuleitungen 1a, 1b, 1c auf. Bevorzugt weist der stabförmige Isolierkörper 3 mindestens so viele Durchgänge auf, wie die Anordnung Zuleitungen 1a, 1b, 1c umfasst. Ferner kann die Anzahl an Durchgängen durch den stabförmigen Isolierkörper 3 die Anzahl an Zuleitungen 1a, 1b, 1c übersteigen. Mit einer Anzahl an Durchgängen durch die Isolierung 3, welche die Anzahl an Zuleitungen 1a, 1b, 1c übersteigt, können zu einem späteren Zeitpunkt weitere Zuleitungen hinzugefügt werden. Ferner ist es möglich, dass derselbe stabförmige Isolierkörper 3 für Ausführungsformen mit unterschiedlichen Anzahlen an Zuleitungen 1a, 1b, 1c verwendet wird. Durch den Einsatz einer stabförmigen Isolierung 3 mit einer vorbestimmten Anzahl an Durchgängen, welche alle Ausführungsformen abdeckt, wird die Variantenvielfalt begrenzt. Es sinkt damit die Wahrscheinlichkeit dafür, dass eine der Varianten im Laufe ihres Betriebes ausfällt.

Der oder die Durchgänge durch den stabförmigen Isolierkörper 3 haben jeweils einen Durchmesser. Beispielsweise können der oder die Durchgänge durch den stabförmigen Isolierkörper 3 Durchmesser von mindestens 0.6 Millimetern oder von mindestens 1.1 Millimetern aufweisen. Es sind auch einer oder mehrere Durchgänge durch den stabförmigen Isolierkörper 3 möglich, deren Durchmesser grösser als 1.5 Millimeter sind.

Im Falle mehrerer Durchgänge durch den stabförmigen Isolierkörper 3 weisen vorzugweise mindestens zwei Durchgänge durch den stabförmigen Isolierkörper 3 gleiche Durchmesser auf. Besonders bevorzugt weisen alle Durchgänge durch den stabförmigen Isolierkörper 3 gleiche Durchmesser auf. Gleiche Durchmesser der Durchgänge durch den stabförmigen Isolierkörper 3 führen zu einer geringen Variantenvielfalt. Es sinkt damit die Wahrscheinlichkeit dafür, dass eine der Varianten im Laufe ihres Betriebes ausfällt. Zudem sinkt der Aufwand bei der Herstellung der stabförmigen Isolierung 3.

Mindestens ein Durchgang durch den stabförmigen Isolierkörper 3 kann eine Bohrung, beispielsweise eine Bohrung mit rundem Querschnitt, umfassen. Insbesondere kann mindestens ein Durchgang durch den stabförmigen Isolierkörper 3 eine Bohrung, beispielsweise eine Bohrung mit rundem Querschnitt, sein. Ferner können sämtliche Durchgänge durch den stabförmigen Isolierkörper 3 jeweils eine Bohrung, beispielsweise eine Bohrung mit rundem Querschnitt, umfassen. Zudem können sämtliche Durchgänge durch den stabförmigen Isolierkörper 3 jeweils eine Bohrung, beispielsweise eine Bohrung mit rundem Querschnitt, sein. Die Ausgestaltung der Durchgänge durch den stabförmigen Isolierkörper 3 als Bohrungen ermöglicht den Einsatz von Standardwerkzeugen zur Herstellung des Isolierkörpers 3.

Die Ummantelung 2 weist ein erstes 4a und ein zweites Ende 4b auf. Das erste Ende 4a der Ummantelung 2 ist verschieden vom zweiten Ende 4b der Ummantelung 2. Das erste Ende 4a der Ummantelung 2 liegt dem zweiten Ende 4b der Ummantelung 2 gegenüber.

Die Ummantelung 2 weist an ihrem ersten Ende 4a eine erste Öffnung auf. Die erste Öffnung hat einen vorzugsweise runden Querschnitt. Die Ummantelung 2 weist an ihrem zweiten Ende 4b eine zweite Öffnung auf. Die zweite Öffnung hat einen vorzugsweise runden Querschnitt. Die erste Öffnung der Ummantelung 2 ist verschieden von der zweiten Öffnung der Ummantelung 2. In einer Ausführungsform haben sowohl die erste Öffnung der Ummantelung 2 als auch die zweite Öffnung der Ummantelung 2 einen runden Querschnitt.

Die Fläche des Querschnittes der ersten Öffnung der Ummantelung 2 ist meist verschieden von der Fläche des Querschnittes der zweiten Öffnung der Ummantelung 2. Insbesondere ist die geringste Fläche des Querschnittes der ersten Öffnung der Ummantelung 2 meist verschieden von der geringsten Fläche des Querschnittes der zweiten Öffnung. Es sind jedoch Ausführungen möglich, in welchen die Flächen der Querschnitte der ersten und der zweiten Öffnung der Ummantelung 2 gleich sind. Insbesondere können die geringsten Flächen der Querschnitte der ersten und der zweiten Öffnung der Ummantelung 2 gleich sein.

In Richtung des zweiten Endes 4b der Ummantelung 2 ist ein Einfasskörper 5 angeordnet. Der Einfasskörper 5 grenzt an den stabförmigen Isolierkörper 3 und an die Ummantelung 2. Der Einfasskörper 5 weist eine erste Oberfläche auf, welche an eine Fläche an der Innenseite der Ummantelung 2 grenzt. Der Einfasskörper 5 weist eine zweite Oberfläche auf, welche an eine Fläche an der Aussenseite des stabförmigen Isolierkörpers 3 grenzt. Vorzugsweise ist die erste Oberfläche des Einfasskörpers 5 zylinderförmig. Vorzugsweise ist die zweite Oberfläche des Einfasskörpers 5 zylinderförmig. In einer Ausführungsform sind die erste und die zweite Oberfläche des Einfasskörpers 5 zylinderförmig.

Ein geringster Abstand zwischen der ersten und der zweiten Oberfläche des Einfasskörpers 5 beeinflusst einen Abstand des stabförmigen Isolierkörpers 3 von der Ummantelung 2. Der Abstand des stabförmigen Isolierkörpers 3 von der Ummantelung 2 kann beispielsweise weniger als zehn Millimeter oder weniger als fünf Millimeter betragen. Ein Abstand zwischen dem stabförmigen Isolierkörper 3 und der Ummantelung 2 erleichtert die Herstellung der Anordnung. Ein Abstand zwischen dem stabförmigen Isolierkörper 3 und Ummantelung 2 ermöglicht eine (begrenzte) thermische Isolation des Isolierkörpers 3 und der Zuleitungen 1a, 1b, 1c von der Ummantelung 2. Eine solche (begrenzte) thermische Isolation ist vorteilhaft im Hinblick auf die erhöhten Temperaturen im Abgaskanal einer Verbrennungsvorrichtung.

In einer Ausführungsform ist der Einfasskörper 5 zumindest teilweise aus einer Vergussmasse gefertigt. Beispielsweise kann der Einfasskörper 5 ganz oder teilweise aus einem wärmebeständigen Epoxidharz hergestellt sein. In einer anderen Ausführungsform ist der Einfasskörper 5 aus Polytetrafluorethylen gefertigt.

Ein Einfasskörper 5 als Vergusskörper trägt zu einer gasdichten Anordnung bei.

Der Einfasskörper 5 oder Teile des Einfasskörpers 5 werden nach einem Aspekt der vorliegenden Offenbarung mit einem additiven Herstellungsverfahren wie dem dreidimensionalen Druck hergestellt. Die Herstellung des Einfasskörpers 5 oder von Teilen des Einfasskörper 5 kann in einer speziellen Ausführungsform durch selektives Lasersintern erfolgen.

Der Einfasskörper 5 weist vorzugsweise einen spezifischen elektrischen Widerstand ρ von mindestens einem Megaohm · Zentimeter bei Temperaturen von 393 Kelvin auf:

$$\rho > 1\ M\Omega \cdot cm \text{ bei } 393 \text{ Kelvin}$$

Besonders bevorzugt ist der spezifische elektrische Widerstand ρ bei Temperaturen von 393 Kelvin grösser als fünf Megaohm · Zentimeter:

$$\rho > 5\ M\Omega \cdot cm \text{ bei } 393 \text{ Kelvin}$$

Weiterhin bevorzugt ist der spezifische elektrische Widerstand ρ bei Temperaturen von 393 Kelvin grösser als zehn Megaohm · Zentimeter:

$$\rho > 10\ M\Omega \cdot cm \text{ bei } 393 \text{ Kelvin}$$

Hohe spezifische Widerstände ermöglichen eine ausreichende elektrische Isolierung zwischen den Zuleitungen 1a, 1b, 1c. Jedwedes Signal, das aus einem an die Zuleitungen 1a, 1b, 1c angeschlossenen Sensorelement gewonnen wird, wird mithin wenig verfälscht.

Der Einfasskörper 5 weist mindestens einen Durchgang für eine der Zuleitungen 1a, 1b, 1c auf. Bevorzugt weist der Einfasskörper 5 mindestens so viele Durchgänge auf, wie die Anordnung Zuleitungen 1a, 1b, 1c umfasst. Ferner kann die Anzahl an Durchgängen durch den Einfasskörper 5 die Anzahl an Zuleitungen 1a, 1b, 1c übersteigen. Mit einer Anzahl an Durchgängen durch die Isolierung 3, welche die Anzahl an Zuleitungen 1a, 1b, 1c übersteigt, können zu einem späteren Zeitpunkt weitere Zuleitungen hinzugefügt werden. Ferner ist es möglich, dass derselbe Einfasskörper 5 für Ausführungsformen mit unterschiedlichen Anzahlen an Zuleitungen 1a, 1b, 1c verwendet wird. Durch den Einsatz eines Einfasskörpers 5 mit einer vorbestimmten Anzahl an Durchgängen, welche alle Ausführungsformen abdeckt, wird die Variantenvielfalt begrenzt. Es sinkt damit die Wahrscheinlichkeit dafür, dass eine der Varianten im Laufe ihres Betriebes ausfällt.

Der oder die Durchgänge durch den Einfasskörper 5 haben jeweils einen Durchmesser. Beispielsweise können der oder die Durchgänge durch den Einfasskörper 5 Durchmesser von mindestens 0.6 Millimetern oder von mindestens 1.1 Millimetern aufweisen. Es sind auch einer oder mehrere Durchgänge durch den Einfasskörper 5 möglich, deren Durchmesser grösser als 1.5 Millimeter sind.

Im Falle mehrerer Durchgänge durch den Einfasskörper 5 weisen vorzugweise mindestens zwei Durchgänge durch den Einfasskörper 5 gleiche Durchmesser auf. Besonders bevorzugt weisen alle Durchgänge durch den Einfasskörper 5 gleiche Durchmesser auf. Gleiche Durchmesser der Durchgänge durch den Einfasskörper 5 führen zu einer geringen Variantenvielfalt. Es sinkt damit die Wahrscheinlichkeit dafür, dass eine der Varianten im Laufe ihres Betriebes ausfällt. Zudem sinkt der Aufwand bei der Herstellung des Einfasskörpers 5.

Speziell bevorzugt weisen der stabförmige Isolierkörper 3 und der Einfasskörper 5 gleiche Anzahlen an Durchgängen auf. Die Durchgänge durch den stabförmigen Isolierkörper 3 und den Einfasskörper 5 haben idealerweise auch die gleichen oder im Wesentlichen die gleichen Durchmesser. Im Wesentlichen die gleichen Durchmesser bedeutet hier, dass die Durchmesser bis auf Fertigungstoleranzen gleich sind. Dabei sind die Durchgänge durch den stabförmigen Isolierkörper 3 und durch den Einfasskörper 5 so angeordnet, dass mindestens eine Zuleitung 1a direkt durch die Anordnung hindurch verläuft. In einer Ausführungsform sind die jeweiligen Durchgänge so angeordnet, dass alle Zuleitungen 1a, 1b, 1c direkt durch die Anordnung hindurch verlaufen. Ein direkter Verlauf durch die Anordnung ermöglicht einen geradlinigen Durchgang durch den stabförmigen Isolierkörper 3 und durch den Einfassköper 5 ohne Knickstellen.

Darüber hinaus können die Durchgänge durch den stabförmigen Isolierkörper 3 und den Einfasskörper 5 auch ähnliche Durchmesser aufweisen. Beispielsweise können die Durchgänge durch den Einfasskörper 5 jeweils ein wenig breiter sein als diejenigen Durchgänge durch den stabförmigen Isolierkörper 3, um eine einfachere Montage zu ermöglichen. Dabei sind die Durchgänge durch den stabförmigen Isolierkörper 3 und durch den Einfasskörper 5 so angeordnet, dass mindestens eine Zuleitung 1a direkt durch die Anordnung hindurch verläuft. In einer Ausführungsform sind die jeweiligen Durchgänge so angeordnet, dass alle Zuleitungen 1a, 1b, 1c direkt durch die Anordnung hindurch verlaufen.

Ein direkter Verlauf durch die Anordnung ermöglicht einen geradlinigen Durchgang durch den stabförmigen Isolierkörper 3 und durch den Einfassköper 5 ohne Knickstellen.

Mindestens ein Durchgang durch den Einfasskörper 5 kann eine Bohrung, beispielsweise eine Bohrung mit rundem Querschnitt, umfassen. Insbesondere kann mindestens ein Durchgang durch den Einfasskörper 5 eine Bohrung, beispielsweise eine Bohrung mit rundem Querschnitt, sein. Ferner können sämtliche Durchgänge durch den Einfasskörper 5 jeweils eine Bohrung, beispielsweise eine Bohrung mit rundem Querschnitt, umfassen. Zudem können sämtliche Durchgänge durch den Einfasskörper 5 jeweils eine Bohrung, beispielsweise eine Bohrung mit rundem Querschnitt, sein. Die Ausgestaltung der Durchgänge durch den Einfasskörper 5 als Bohrungen ermöglicht den Einsatz von Standardwerkzeugen zur Herstellung des Einfassköpers 5.

In dem Einfasskörper 5 ist eine Aussparung für einen ersten Ring 6a, 6b vorgesehen. Der erste Ring 6a, 6b umrundet den stabförmigen Isolierkörper 3. Der erste Ring 6a, 6b liegt an dem stabförmigen Isolierkörper 3 an. Ferner liegt der erste Ring 6a, 6b an der Ummantelung 2 an. Mithin liegt der erste Ring 6a, 6b sowohl an dem stabförmigen Isolierkörper 3 als auch an der Ummantelung 2 an. Der erste Ring 6a, 6b befindet sich zwischen dem stabförmigen Isolierkörper 3 und der Ummantelung 2. Beispielsweise kann der erste Ring 6a, 6b einen Abschnitt umfassen. Der Abschnitt des ersten Ringes 6a, 6b kann in einer ersten Rille oder in einer ersten Furche, welche durch den Einfasskörper 5 und/oder die Ummantelung 2 gebildet wird, verlaufen.

In einer Ausführungsform ist der erste Ring 6a, 6b ein O-Ring wie beispielsweise ein temperaturbeständiger O-Ring. Insbesondere kann der O-Ring aus einem Silikonpolymer gefertigt sein. In einer weiteren Ausführungsform ist der erste Ring 6a, 6b ein Haltering wie beispielsweise ein temperaturbeständiger Haltering. Insbesondere kann der Haltering aus einem Silikonpolymer gefertigt sein. In wiederum einer weiteren Ausführungsform ist der erste Ring 6a, 6b ein O-Ring und Haltering wie beispielsweise ein temperaturbeständiger O-Ring und Haltering. Insbesondere kann der O-Ring und Haltering aus einem Silikonpolymer gefertigt sein.

Der erste Ring 6a, 6b kann darüber hinaus ein Dichtring sein. Das heisst, dass der erste Ring 6a, 6b dazu beiträgt, dass keine Gase zwischen den Enden 4a, 4b durchgeleitet werden. Beispielsweise kann der erste Ring 6a, 6b dazu beitragen, dass keine Verbrennungsgase zwischen den Enden 4a, 4b der Anordnung durchgeleitet werden. In einer besonderen Ausführungsform ist der erste Ring 6a, 6b sowohl O-Ring als auch Dichtring. In einer weiteren speziellen Ausführungsform ist der erste Ring 6a, 6b sowohl O-Ring als auch Haltering als auch Dichtring.

Der erste Ring 6a, 6b ist in der Nähe des zweiten Endes 4b der Anordnung angeordnet. Das heisst, dass der erste Ring 6a, 6b einen geringsten Abstand von dem ersten Ende 4a und einen geringsten Abstand von dem zweiten Ende 4b aufweist. Dabei ist der geringste Abstand des ersten Rings 6a, 6b von dem ersten Ende 4a grösser als dessen geringster Abstand von dem zweiten Ende 4b.

Näher an dem ersten Ende 4a ist ein zweiter Ring 7a, 7b angeordnet. Das heisst, dass der zweite Ring 7a, 7b einen geringsten Abstand von dem ersten Ende 4a und einen geringsten Abstand von dem zweiten Ende 4b aufweist. Dabei ist der geringste Abstand des zweiten Rings 7a, 7b von dem ersten Ende 4a kleiner als dessen geringster Abstand von dem zweiten Ende 4b. Der geringste Abstand des zweiten Rings 7a, 7b von dem ersten Ende 4a ist kleiner als derjenige Abstand des ersten Rings 6a, 6b von demselben Ende 4a. Der geringste Abstand des zweiten Rings 7a, 7b von dem zweiten Ende 4b ist grösser als derjenige Abstand des ersten Rings 6a, 6b von demselben Ende 4b.

Der zweite Ring 7a, 7b umrundet den stabförmigen Isolierkörper 3. Der zweite Ring 7a, 7b liegt an dem stabförmigen Isolierkörper 3 an. Ferner liegt der zweite Ring 7a, 7b an der Ummantelung 2 an. Mithin liegt der zweite Ring 7a, 7b sowohl an dem stabförmigen Isolierkörper 3 als auch an der Ummantelung 2 an. Beispielsweise kann der zweite Ring 7a, 7b einen Abschnitt umfassen. Der Abschnitt des zweiten Ringes 7a, 7b kann in einer zweiten Rille oder in einer zweiten Furche, welche durch die Ummantelung 2 gebildet wird, verlaufen. Der zweite Ring 7a, 7b befindet sich demnach zwischen dem stabförmigen Isolierkörper 3 und der Ummantelung 2.

In einer Ausführungsform ist der zweite Ring 7a, 7b ein Sprengring wie beispielsweise ein temperaturbeständiger Sprengring. Insbesondere kann der Sprengring aus einem korrosionsbeständigen Stahl gefertigt sein. In einer weiteren Ausführungsform ist der zweite Ring 7a, 7b ein Haltering wie beispielsweise ein temperaturbeständiger Haltering. Insbesondere kann der Haltering aus einem korrosionsbeständigen Stahl gefertigt sein. In wiederum einer weiteren Ausführungsform ist der zweite Ring 7a, 7b ein Sprengring und Haltering wie beispielsweise ein temperaturbeständiger Sprengring und Haltering. Insbesondere kann der Sprengring und Haltering aus einem korrosionsbeständigen Stahl gefertigt sein.

Der erste Ring 6a, 6b und der zweite Ring 7a, 7b tragen zur Fixierung des stabförmigen Isolierkörpers 3 gegenüber der Ummantelung 2 bei. Insbesondere ermöglichen der erste Ring 6a, 6b und der zweite Ring 7a, 7b den vorgenannten geringsten Abstand zwischen dem stabförmigen Isolierkörper 3 und der Ummantelung 2. Ferner hindern erste Ring 6a, 6b und der zweite Ring 7a, 7b eine laterale Bewegung des stabförmigen Isolierkörpers 3 zwischen den Enden 4a, 4b.

Der stabförmige Isolierkörper 3 und die Ummantelung 2 können unterschiedliche Temperaturausdehnungskoeffizienten aufweisen. Beispielsweise kann der stabförmige Isolierkörper 3 einen Temperaturausdehnungskoeffizienten $\alpha$ zwischen $2 \cdot 10^{-6}$/Kelvin und $10 \cdot 10^{-6}$/Kelvin im Bereich zwischen 313 Kelvin und 673 Kelvin aufweisen:

$$2 \cdot 10^{-6}/\mathrm{Kelvin} < \alpha < 10 \cdot 10^{-6}/\mathrm{Kelvin}$$

Insbesondere kann der stabförmige Isolierkörper 3 einen Temperaturausdehnungskoeffizienten $\alpha$ zwischen $3 \cdot 10^{-6}$/Kelvin und $9 \cdot 10^{-6}$/Kelvin im Bereich zwischen 313 Kelvin und 673 Kelvin aufweisen:

$$3 \cdot 10^{-6}/\mathrm{Kelvin} < \alpha < 9 \cdot 10^{-6}/\mathrm{Kelvin}$$

Besonders bevorzugt kann der stabförmige Isolierkörper 3 einen Temperaturausdehnungskoeffizienten $\alpha$ zwischen $4 \cdot 10^{-6}$/Kelvin und $8 \cdot 10^{-6}$/Kelvin im Bereich zwischen 313 Kelvin und 673 Kelvin aufweisen:

$$4 \cdot 10^{-6}/\mathrm{Kelvin} < \alpha < 8 \cdot 10^{-6}/\mathrm{Kelvin}$$

Die vorgenannten Temperaturausdehnungskoeffizienten $\alpha$ beziehen sich auf eine Achse, welche durch mindestens eine der Zuleitungen 1a, 1b, 1c definiert ist. Das heisst, dass es sich bei diesen Temperaturausdehnungskoeffizienten $\alpha$ um axiale Koeffizienten entlang der vorgenannten Achse handelt.

Zudem kann die Ummantelung 2 einen Temperaturausdehnungskoeffizienten $\alpha$ zwischen $10.10^{-6}$/Kelvin und $20 \cdot 10^{-6}$/Kelvin bei 293 Kelvin aufweisen:

$$10 \cdot 10^{-6}/\mathrm{Kelvin} < \alpha < 20 \cdot 10^{-6}/\mathrm{Kelvin}$$

Insbesondere kann die Ummantelung 2 einen Temperaturausdehnungskoeffizienten $\alpha$ zwischen $11 \cdot 10^{-6}$/Kelvin und $19 \cdot 10^{-6}$/Kelvin bei 293 Kelvin aufweisen:

$$11 \cdot 10^{-6}/\mathrm{Kelvin} < \alpha < 19 \cdot 10^{-6}/\mathrm{Kelvin}$$

Besonders bevorzugt kann die Ummantelung 2 einen Temperaturausdehnungskoeffizienten $\alpha$ zwischen $11 \cdot 10^{-6}$/Kelvin und $18 \cdot 10^{-6}$/Kelvin bei 293 Kelvin aufweisen:

$$11 \cdot 10^{-6}/\mathrm{Kelvin} < \alpha < 18 \cdot 10^{-6}/\mathrm{Kelvin}$$

Die vorgenannten Temperaturausdehnungskoeffizienten $\alpha$ beziehen sich auf eine Achse, welche durch mindestens eine der Zuleitungen 1a, 1b, 1c definiert ist. Das heisst, dass es sich bei diesen Temperaturausdehnungskoeffizienten $\alpha$ um axiale Koeffizienten entlang der vorgenannten Achse handelt.

Infolge der unterschiedlichen Temperaturausdehnungskoeffizienten der Ummantelung 2 und des stabförmigen Isolierkörpers 3 kann es zu mechanischen Spannungen in der Anordnung kommen. Das Problem der mechanischen Spannungen wird verschärft durch den weiten Bereich an Temperaturen, welchen die Anordnung im Betrieb ausgesetzt ist. Der erste 6a, 6b und der zweite Ring 7a, 7b lagern den stabförmigen Isolierkörper 3 gegenüber der Ummantelung 2 so, dass Risse infolge dieser mechanischen Spannungen vermieden werden.

Optional ist noch näher an dem ersten Ende 4a ein dritter Ring 8a, 8b angeordnet. Das heisst, dass der dritte Ring 8a, 8b einen geringsten Abstand von dem ersten Ende 4a und einen geringsten Abstand von dem zweiten Ende 4b aufweist. Dabei ist der geringste Abstand des dritten Rings 8a, 8b von dem ersten Ende 4a kleiner als dessen geringster Abstand von dem zweiten Ende 4b. Der geringste Abstand des dritten Rings 8a, 8b von dem ersten Ende 4a ist kleiner als derjenige Abstand des ersten Rings 6a, 6b von demselben Ende 4a. Der geringste Abstand des dritten Rings 8a, 8b von dem zweiten Ende 4b ist grösser als derjenige Abstand des ersten Rings 6a, 6b von demselben Ende 4b.

Der geringste Abstand des dritten Rings 8a, 8b von dem ersten Ende 4a ist kleiner als derjenige Abstand des zweiten Rings 7a, 7b von demselben Ende 4a. Der geringste Abstand des dritten Rings 8a, 8b von dem zweiten Ende 4b ist grösser als derjenige Abstand des zweiten Rings 7a, 7b von demselben Ende 4b.

Der optionale dritte Ring 8a, 8b umrundet den stabförmigen Isolierkörper 3. Der dritte Ring 8a, 8b liegt nicht an dem stabförmigen Isolierkörper 3 an. Das heisst, dass der dritte Ring 8a, 8b und der stabförmige Isolierkörper 3 einander nicht berühren. Der dritte Ring 8a, 8b und der stabförmige Isolierkörper 3 sind voneinander beabstandet. Der dritte Ring 8a, 8b liegt an der Ummantelung 2 an. Beispielsweise kann der dritte Ring 8a, 8b einen Abschnitt umfassen. Der Abschnitt des dritten Ringes 8a, 8b kann in einer dritten Rille oder in einer dritten Furche, welche durch die Ummantelung 2 gebildet wird, verlaufen. Der dritte Ring 8a, 8b befindet sich demnach zwischen dem stabförmigen Isolierkörper 3 und der Ummantelung 2.

In einer Ausführungsform ist der dritte Ring 8a, 8b ein Sprengring wie beispielsweise ein temperaturbeständiger Sprengring. Insbesondere kann der Sprengring aus einem korrosionsbeständigen Stahl gefertigt sein. In einer weiteren Ausführungsform ist der dritte Ring 8a, 8b ein Haltering wie beispielsweise ein temperaturbeständiger Haltering. Insbesondere kann der Haltering aus einem korrosionsbeständigen Stahl gefertigt sein. In wiederum einer weiteren Ausführungsform ist der dritte Ring 8a, 8b ein Sprengring und Haltering wie beispielsweise ein temperaturbeständiger Sprengring und Haltering. Insbesondere kann der Sprengring und Haltering aus einem korrosionsbeständigen Stahl gefertigt sein.

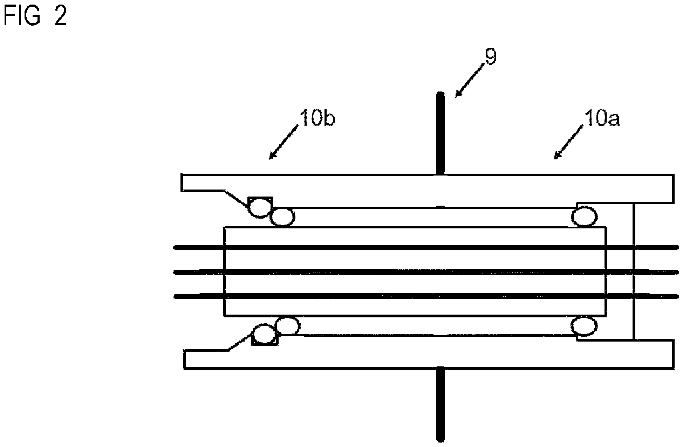

FIG 2 zeigt die Anordnung mit den Zuleitungen 1a, 1b, 1c aus FIG 1 eingebaut in eine Wand 9. Dabei ist die Anordnung mit den Zuleitungen 1a, 1b, 1c unterteilt in einen ersten Abschnitt 10a und einen zweiten Abschnitt 10b. Die Anordnung bildet so eine Durchführung für die Zuleitungen 1a, 1b, 1c durch die Wand 9.

Der erste Abschnitt 10a befindet sich ausserhalb eines Abgaskamins einer Verbrennungsvorrichtung und ausserhalb eines Rauchgaskamins der Verbrennungsvorrichtung und/oder ausserhalb eines Schornsteins der Verbrennungsvorrichtung. Der erste Abschnitt 10a befindet sich auch ausserhalb eines Feuerraumes der Verbrennungsvorrichtung.

Vorzugsweise ist der erste Abschnitt 10a umgeben von Umgebungsluft mit einer Temperatur zwischen 263 Kelvin und 318 Kelvin, insbesondere zwischen 273 Kelvin und 308 Kelvin. Besonders bevorzugt ist der erste Abschnitt 10a umgeben von Umgebungsluft mit einer Temperatur zwischen 283 Kelvin und 298 Kelvin. Das heisst, dass der erste Abschnitt 10a umgeben ist von Luft mit einer Temperatur in der Nähe der Raumtemperatur.

Der erste Abschnitt 10a ist ferner umgeben von Luft mit einem Druck zwischen 1100 Hektopascal und 700 Hektopascal, insbesondere zwischen 1100 Hektopascal und 800 Hektopascal. Besonders bevorzugt ist der erste Abschnitt 10a umgeben von Umgebungsluft mit einem Druck zwischen 1050 Hektopascal und 900 Hektopascal. Das heisst, dass der erste Abschnitt 10a umgeben ist von Luft mit einem Druck in der Nähe der Normalbedingungen.

Der zweite Abschnitt 10b befindet sich innerhalb einer Struktur ausgewählt aus:

einem Abgaskamins einer Verbrennungsvorrichtung,
einem Rauchgaskamin der Verbrennungsvorrichtung,
einem Schornstein der Verbrennungsvorrichtung.

Der zweite Abschnitt 10b kann sich auch innerhalb zweier Strukturen ausgewählt aus:

einem Abgaskamins einer Verbrennungsvorrichtung,
einem Rauchgaskamin der Verbrennungsvorrichtung,
einem Schornstein der Verbrennungsvorrichtung befinden. Beispielsweise kann ein und dieselbe Struktur zugleich als Abgaskamin einer Verbrennungsvorrichtung und als Rauchgaskamin der Verbrennungsvorrichtung dienen. Das heisst, dass die Struktur den Abgaskamin und den Rauchgaskamin der Verbrennungsvorrichtung umfasst.

Zudem kann ein und dieselbe Struktur zugleich als Abgaskamin der Verbrennungsvorrichtung und als Schornstein der Verbrennungsvorrichtung dienen. Das heisst, dass die Struktur den Abgaskamin und den Schornstein der Verbrennungsvorrichtung umfasst.

Darüber hinaus kann ein und dieselbe Struktur zugleich als Rauchgaskamin der Verbrennungsvorrichtung und als Schornstein der Verbrennungsvorrichtung dienen. Das heisst, dass die Struktur den Rauchgaskamin und den Schornstein der Verbrennungsvorrichtung umfasst.

Der zweite Abschnitt 10b kann sich ferner innerhalb dreier Strukturen, nämlich innerhalb eines Abgaskamins einer Verbrennungsvorrichtung und innerhalb eines Rauchgaskamins der Verbrennungsvorrichtung und innerhalb eines Schornsteins der Verbrennungsvorrichtung befinden. Beispielsweise kann ein und dieselbe Struktur zugleich als Abgaskamin einer Verbrennungsvorrichtung und als Rauchgaskamin der Verbrennungsvorrichtung und als Schornstein der Verbrennungsvorrichtung dienen. Das heisst, dass die Struktur den Abgaskamin und den Rauchgaskamin und den Schornstein der Verbrennungsvorrichtung umfasst.

Der zweite Abschnitt 10b kann sich weiterhin innerhalb eines Feuerraumes einer Verbrennungsvorrichtung befinden.

Vorzugsweise ist der zweite Abschnitt 10b umgeben von Abgasen und/oder Rauchgasen mit einer Temperatur bis zu 873 Kelvin, insbesondere bis zu 673 Kelvin. Besonders bevorzugt ist der zweite Abschnitt 10b der Anordnung umgeben von Abgasen und/oder Rauchgasen mit einer Temperatur von bis zu 500 Kelvin. Das heisst, dass der zweite Abschnitt 10b umgeben ist von Abgasen und/oder Rauchgasen, welche aus einem Feuerraum einer Verbrennungsvorrichtung stammen.

Im Allgemeinen liegt innerhalb des zweiten Abschnittes 10b ein erster Temperaturgradient vor. Das heisst, dass die Temperaturen der Abgase und/oder Rauchgase mit zunehmender Entfernung vom Feuerraum der Verbrennungsvorrichtung abnehmen. Im Allgemeinen liegt innerhalb des zweiten Abschnittes 10b auch ein zweiter Temperaturgradient vor. Das heisst, dass die Temperaturen der Abgase und/oder Rauchgase in Richtung der Wand abnehmen.

Die Abgase und/oder Rauchgase weisen im zweiten Abschnitt 10b Drücke auf, welche gleich oder ähnlich den Drücken im ersten Abschnitt 10a sind. Ähnliche Drücke bedeutet, dass die Drücke sich um weniger als 100 Hektopascal, bevorzugt um weniger als 50 Hektopascal, besonders bevorzugt um weniger als 20 Hektopascal unterscheiden. Tiefe Druckunterschiede verringern die mechanische Belastung der Wand 9 und der Anordnung umfassend den ersten 10a und den zweiten Abschnitt 10b.

Die Wand 9 und die Ummantelung 2 sind beispielsweise metallisch, vorzugsweise aus Stahl, insbesondere aus korrosionsbeständigem Stahl, gefertigt. Die Wand 9 und die Ummantelung 2 sind im Betrieb einem weiten Bereich an Temperaturen ausgesetzt. Infolge unterschiedlicher Temperaturausdehnungskoeffizienten $\alpha$ zwischen der Wand 9 und der Ummantelung 2 kann es so zu Rissen kommen.

Aussen an der Ummantelung 2 kann eine Markierung angebracht sein, welche für einen Monteur eine Einbautiefe in die Wand 9 einer Verbrennungsvorrichtung angibt. Insbesondere kann aussen an der Ummantelung 2 eine Markierung angebracht sein, welche für einen Monteur eine Einbautiefe in eine Aussenwand 9 einer Verbrennungsvorrichtung angibt. Ferner kann aussen an der Ummantelung 2 eine Skala angebracht sein, welche für einen Monteur eine Einbautiefe in eine Aussenwand 9 einer Verbrennungsvorrichtung angibt. Die Skala umfasst mehrere Werte und der Monteur wählt so eine geeignete Einbautiefe.

Zur Vermeidung mechanischer Spannungen werden die Wand 9 und die Ummantelung 2 vorzugsweise aus Materialien mit ähnlichen Temperaturausdehnungskoeffizienten $\alpha$ gefertigt. Beispielsweise können die Wand 9 und die Ummantelung 2 einen Temperaturausdehnungskoeffizienten $\alpha$ zwischen $10.10^{-6}$/Kelvin und $20.10^{-6}$/Kelvin bei 293 Kelvin aufweisen:

$$10 \cdot 10^{-6}/\mathrm{Kelvin} < \alpha < 20 \cdot 10^{-6}/\mathrm{Kelvin}$$

Insbesondere können die Wand 9 und die Ummantelung 2 einen Temperaturausdehnungskoeffizienten $\alpha$ zwischen $11 \cdot 10^{-6}$/Kelvin und $19 \cdot 10^{-6}$/Kelvin bei 293 Kelvin aufweisen:

$$11 \cdot 10^{-6}/\mathrm{Kelvin} < \alpha < 19 \cdot 10^{-6}/\mathrm{Kelvin}$$

Besonders bevorzugt können die Wand 9 und die Ummantelung 2 einen Temperaturausdehnungskoeffizienten $\alpha$ zwischen $11 \cdot 10^{-6}$/Kelvin und $18 \cdot 10^{-6}$/Kelvin bei 293 Kelvin aufweisen:

$$11 \cdot 10^{-6}/\mathrm{Kelvin} < \alpha < 18 \cdot 10^{-6}/\mathrm{Kelvin}$$

Die vorgenannten Temperaturausdehnungskoeffizienten $\alpha$ beziehen sich auf eine Achse, welche durch mindestens eine der Zuleitungen 1a, 1b, 1c definiert ist. Das heisst, dass es sich bei diesen Temperaturausdehnungskoeffizienten $\alpha$ um axiale Koeffizienten entlang der vorgenannten Achse handelt.

Gemäss einem Aspekt der vorliegenden Offenbarung ist die Ummantelung 2 in die Wand 9 eingeschraubt. Gemäss einem speziellen Aspekt der vorliegenden Offenbarung ist die Ummantelung 2 in die Aussenwand 9 eingeschraubt.

In einer weiteren Ausführungsform ist zwischen der Ummantelung 2 und der Wand 9 eine Schicht zur thermischen Entkopplung vorgesehen. Beispielsweise ist zwischen der Ummantelung 2 und einer Aussenwand 9 der Verbrennungsvorrichtung eine Schicht zur thermischen Entkopplung vorgesehen. Die Schicht zur thermischen Entkopplung kann insbesondere einen glasfaserverstärten Kunststoff und/oder ein keramisches Material umfassen. In einer Ausführungsform besteht die Schicht zur thermischen Entkopplung aus einem glasfaserverstärten Kunststoff und/oder aus einem keramischen Material.

Mit anderen Worten, die vorliegende Offenbarung lehrt eine Anordnung umfassend mindestens eine erste elektrisch leitfähige Zuleitung (1a, 1b, 1c), eine Ummantelung (2) und einen stabförmigen Isolierkörper (3);

> wobei die Ummantelung (2) ein erstes Ende (4a) und ein zweites Ende (4b) aufweist und das erste Ende (4a) verschieden von dem zweiten Ende (4b) ist und das erste Ende (4a) dem zweiten Ende (4b) gegenüberliegt und die Ummantelung (2) eine erste Öffnung an dem ersten Ende (4a) und eine zweite Öffnung an dem zweiten Ende (4b) aufweist;
>
> wobei die mindestens eine erste Zuleitung (1a, 1b, 1c) von dem ersten Ende (4a) zum zweiten Ende (4b) durch die Anordnung hindurch verläuft und eine Achse definiert;
>
> wobei der stabförmige Isolierkörper (3) mindestens einen Durchgang durch den stabförmigen Isolierkörper (3) umfasst und ein erster Abschnitt der mindestens einen Zuleitung (1a, 1b, 1c) in dem mindestens einen Durchgang durch den stabförmigen Isolierkörper (3) verläuft;
>
> wobei die mindestens eine erste Zuleitung (1a, 1b, 1c) zwischen der ersten Öffnung und der zweiten Öffnung eine erste Länge aufweist;
>
> wobei der erste Abschnitt der mindestens einen Zuleitung (1a, 1b, 1c) in dem mindestens einen Durchgang durch den stabförmigen Isolierkörper (3) eine zweite Länge aufweist und die zweite Länge kleiner als die erste Länge ist;
>
> wobei der stabförmige Isolierkörper (3) radial nach aussen von der Achse aus eine Aussenoberfläche umfasst und die Ummantelung (2) radial nach aussen von der Achse aus eine Innenoberfläche umfasst, wobei die Aussenoberfläche des stabförmigen Isolierkörpers (3) und die Innenoberfläche der Ummantelung (2) jeweils parallel zur Achse verlaufen, sodass die Aussenoberfläche des stabförmigen Isolierkörpers (3) und die Innenoberfläche der Ummantelung (2) einander gegenüberliegen; und
>
> wobei die Anordnung einen Spalt mit einer Dicke umfasst, der zwischen der Aussenoberfläche des stabförmigen Isolierkörpers (3) und der Innenoberfläche der Ummantelung (2) angeordnet ist, und Abstandshalter (5, 6a, 6b, 7a, 7b) zur Beabstandung des stabförmigen Isolierkörpers (3) von der Ummantelung (2) umfasst, wobei die Abstandshalter (5, 6a, 6b, 7a, 7b) die Dicke des Spaltes definieren.

Die vorliegende Offenbarung beinhaltet zudem eine der vorgenannten Anordnungen, wobei die Anordnung mindestens eine zweite elektrisch leitfähige Zuleitung (1a, 1b, 1c) umfasst und die mindestens eine zweite elektrisch leitfähige Zuleitung (1a, 1b, 1c) verschieden von der mindestens einen ersten elektrisch leitfähigen Zuleitung (1a, 1b, 1c) ist. Der stabförmige Isolierkörper (3) umfasst mindestens einen weiteren Durchgang und die mindestens eine zweite elektrisch leitfähige Zuleitung (1a, 1b, 1c) verläuft durch den mindestens einen weiteren Durchgang.

Die mindestens eine erste elektrisch leitfähige Zuleitung (1a, 1b, 1c) ist vorzugsweise mindestens eine erste elektrisch leitfähige Zuleitung zu einer Sensoreinheit, insbesondere zu einer Sensoreinheit innerhalb einer Verbrennungsvorrichtung. Die mindestens eine zweite elektrisch leitfähige Zuleitung (1a, 1b, 1c) ist vorzugsweise mindestens eine zweite elektrisch leitfähige Zuleitung zu der Sensoreinheit, insbesondere zu der Sensoreinheit innerhalb der Verbrennungsvorrichtung. Die Sensoreinheit kann beispielsweise innerhalb einer Struktur der Verbrennungsvorrichtung, die Struktur ausgewählt aus:

- einem Abgaskamin,
- einem Rauchgaskamin,
- einem Schornstein

angeordnet sein.

In einer Ausführungsform ist die mindestens eine erste elektrisch leitfähige Zuleitung (1a, 1b, 1c) galvanisch verbunden mit der Sensoreinheit. In einer Ausführungsform sind die mindestens eine erste elektrisch leitfähige Zuleitung (1a, 1b, 1c) und die mindestens eine zweite elektrisch leitfähige Zuleitung (1a, 1b, 1c) galvanisch verbunden mit der Sensoreinheit.

In einer Ausführungsform ist die erste Länge definiert als Distanz zwischen dem ersten (4a) und dem zweiten Ende (4b).

Die vorliegende Offenbarung beinhaltet zudem eine der vorgenannten Anordnungen, wobei der erste Abschnitt der mindestens einen Zuleitung (1a, 1b, 1c) in dem mindestens einen Durchgang durch den stabförmigen Isolierkörper (3) eine zweite Länge aufweist und die zweite Länge kürzer als die erste Länge ist.

Die vorliegende Offenbarung beinhaltet ferner eine der vorgenannten Anordnungen, wobei der erste Abschnitt der mindestens einen Zuleitung (1a, 1b, 1c) in dem mindestens einen Durchgang durch den stabförmigen Isolierkörper (3) eine zweite Länge aufweist und die zweite Länge geringer als die erste Länge ist.

Die vorliegende Offenbarung beinhaltet ferner eine der vorgenannten Anordnungen, wobei die Achse eine Symmetrieachse ist. Die vorliegende Offenbarung beinhaltet ferner eine der vorgenannten Anordnungen, wobei die Achse mittig durch die Anordnung verläuft. In einer Ausführungsform verläuft die Achse von dem ersten Ende (4a) der Anordnung zum zweitem Ende (4b) der Anordnung.

Die vorliegende Offenbarung beinhaltet ausserdem eine der vorgenannten Anordnungen, wobei die Aussenoberfläche des stabförmigen Isolierkörpers (3) und die Innenoberfläche der Ummantelung (2) jeweils entlang der Achse verlaufen.

Die vorliegende Offenbarung beinhaltet weiterhin eine der vorgenannten Anordnungen, wobei die Aussenoberfläche des stabförmigen Isolierkörpers (3) und die Innenoberfläche der Ummantelung (2) von der Achse aus in radialer Richtung beabstandet sind. Vorzugsweise wird damit die Dicke des Spaltes definiert und/oder festgelegt.

Die vorliegende Offenbarung beinhaltet ferner eine der vorgenannten Anordnungen, wobei die Anordnung den Spalt mit einer Dicke umfasst, der zwischen der Aussenoberfläche des stabförmigen Isolierkörpers (3) und der Innenoberfläche der Ummantelung (2) angeordnet ist, und Abstandshalter (5, 6a, 6b, 7a, 7b) zur Beabstandung des stabförmigen Isolierkörpers (3) von der Ummantelung (2) umfasst, wobei die Abstandshalter (5, 6a, 6b, 7a, 7b) die Dicke des Spaltes festlegen.

Der vorgenannte Spalt ist vorzugsweise ein radialer Spalt. In einer Ausführungsform verläuft der Spalt entlang einer geschlossenen Linie (aussen) um den stabförmigen Isolierkörper (3) herum. In einer speziellen Ausführungsform verläuft der Spalt entlang eines Kreises oder entlang einer Ellipse (aussen) um den stabförmigen Isolierkörper (3) herum.

Die vorliegende Offenbarung lehrt ausserdem eine der vorgenannten Anordnungen, wobei die Abstandshalter (5, 6a, 6b, 7a, 7b) zur Beabstandung der Aussenoberfläche des stabförmigen Isolierkörpers (3) von der Innenoberfläche der Ummantelung (2) ausgebildet sind und die Dicke des Spaltes definieren.

Die vorliegende Offenbarung lehrt darüber hinaus eine der vorgenannten Anordnungen, wobei die mindestens eine erste Zuleitung (1a, 1b, 1c) von dem ersten Ende (4a) zum zweiten Ende (4b) geradelinig durch die Anordnung hindurch verläuft und die Achse definiert.

Die vorliegende Offenbarung lehrt ferner eine der vorgenannten Anordnungen,

wobei die Abstandshalter (5, 6a, 6b, 7a, 7b) einen Einfasskörper (5) umfassen; und
wobei der Einfasskörper (5) mindestens einen ersten Abschnitt, der senkrecht zur Achse verläuft und an den stabförmigen Isolierkörper (3) grenzt, umfasst und mindestens einen zweiten Abschnitt, der parallel zur Achse verläuft und an die Ummantelung (2) grenzt, umfasst.

Die vorliegende Offenbarung beinhaltet ferner eine der vorgenannten Anordnungen mit Einfasskörper (5), wobei der zweite Abschnitt des Einfasskörpers (5) verschieden vom ersten Abschnitt des Einfasskörpers (5) ist.

Der Einfasskörper (5) ist verschieden vom stabförmigen Isolierkörper (3). Der Einfasskörper (5) ist in einer Ausführungsform mechanisch und ohne Zerspanung separierbar von dem stabförmigen Isolierkörper (3). Der Einfasskörper (5) ist in einer Ausführungsform mechanisch und zerspanungsfrei separierbar von dem stabförmigen Isolierkörper (3). Der Einfasskörper (5) ist in einer anderen, gasdichten Ausführungsform mechanisch derart mit der Ummantelung (2) und dem stabförmigen Isolierkörper (3) verbunden und/oder vergossen, dass eine Separierung des Einfasskörpers (5) von der Ummantelung (2) und eine Separierung des Einfasskörpers (5) von dem stabförmigen Isolierkörper (3) eine Zerspanung erfordern.

Die vorliegende Offenbarung beinhaltet darüber hinaus eine der vorgenannten Anordnungen mit Einfasskörper (5), wobei der Einfasskörper (5) mindestens einen dritten Abschnitt, der parallel zur Achse verläuft und an die Aussenoberfläche des stabförmigen Isolierkörpers (3) grenzt, umfasst.

Die vorliegende Offenbarung beinhaltet ausserdem eine der vorgenannten Anordnungen mit Einfasskörper (5) und drittem Abschnitt, wobei der dritte Abschnitt des Einfasskörpers (5) verschieden vom zweiten Abschnitt des Einfasskörpers (5) ist. Die vorliegende Offenbarung beinhaltet weiterhin eine der vorgenannten Anordnungen mit Einfasskörper (5) und drittem Abschnitt, wobei der dritte Abschnitt des Einfasskörpers (5) verschieden vom ersten Abschnitt des Einfasskörpers (5) ist.

Die vorliegende Offenbarung lehrt zudem eine der vorgenannten Anordnungen mit Einfasskörper (5), wobei der Einfasskörper (5) mindestens einen Durchgang aufweist und ein zweiter Abschnitt der mindestens einen Zuleitung (1a, 1b, 1c) in dem mindestens einen Durchgang durch den Einfasskörper (5) verläuft.

Die vorliegende Offenbarung lehrt weiterhin eine der vorgenannten Anordnungen mit Einfasskörper (5),
wobei der Einfasskörper (5) auf den stabförmigen Isolierkörper (3) von der Achse aus in axialer Richtung aufsteckbar ist.

Die vorliegende Offenbarung beinhaltet zudem eine der vorgenannten Anordnungen mit Einfasskörper (5), wobei der Einfasskörper (5) an dem stabförmigen Isolierkörper (3) durch Aufstecken in axialer Richtung von der Achse aus befestigbar oder befestigt ist.

Die vorliegende Offenbarung lehrt darüber hinaus eine der vorgenannten Anordnungen mit Einfasskörper (5),
wobei der Einfasskörper (5) in die Ummantelung (2) von der Achse aus in axialer Richtung einsteckbar ist.

Die vorliegende Offenbarung beinhaltet zudem eine der vorgenannten Anordnungen mit Einfasskörper (5), wobei der Einfasskörper (5) an der Ummantelung (2) durch Einstecken in axialer Richtung von der Achse aus befestigbar ist. Die Ummantelung (2) kann dazu ein Befestigungsmittel, welches eine Befestigungsoberfläche, die senkrecht zur Achse verläuft, umfassen.

Die vorliegende Offenbarung lehrt ausserdem eine der vorgenannten Anordnungen mit Einfasskörper (5),
wobei der Einfasskörper (5) einen ersten Abstand zum ersten Ende (4a) aufweist und einen zweiten Abstand zum zweiten Ende (4b) aufweist, wobei der erste Abstand des Einfasskörpers (5) von dem ersten Ende (4a) grösser als der zweite Abstand des Einfasskörpers (5) von dem zweiten Ende (4b) ist.

Die vorliegende Offenbarung beinhaltet ferner eine der vorgenannten Anordnungen mit Einfasskörper (5), wobei der Einfasskörper (5) einen ersten Abstand zum ersten Ende (4a) aufweist und einen zweiten Abstand zum zweiten Ende (4b) aufweist, wobei der erste Abstand des Einfasskörpers (5) von dem ersten Ende (4a) länger als der zweite Abstand des Einfasskörpers (5) von dem zweiten Ende (4b) ist.

Die vorliegende Offenbarung beinhaltet weiterhin eine der vorgenannten Anordnungen mit Einfasskörper (5), wobei der Einfasskörper (5) einen ersten Abstand zur ersten Öffnung der Ummantelung (2) aufweist und einen zweiten Abstand zur zweiten Öffnung der Ummantelung (2) aufweist, wobei der erste Abstand des Einfasskörpers (5) von der ersten Öffnung der Ummantelung (2) grösser als der zweite Abstand des Einfasskörpers (5) von der zweiten Öffnung der Ummantelung (2) ist.

Die vorliegende Offenbarung beinhaltet darüber hinaus eine der vorgenannten Anordnungen mit Einfasskörper (5), wobei der Einfasskörper (5) einen ersten Abstand zur ersten Öffnung der Ummantelung (2) aufweist und einen zweiten Abstand zur zweiten Öffnung der Ummantelung (2) aufweist, wobei der erste Abstand des Einfasskörpers (5) von der ersten Öffnung der Ummantelung (2) länger als der zweite Abstand des Einfasskörpers (5) von der zweiten Öffnung der Ummantelung (2) ist.

Die vorliegende Offenbarung lehrt weiterhin eine der vorgenannten Anordnungen mit Einfasskörper (5),
wobei der stabförmige Isolierkörper (3) und der Einfasskörper (5) jeweils elektrische Isolatoren sind und bei Temperaturen von 393 Kelvin jeweils einen spezifischen elektrischen Widerstand $\rho$ von mindestens einem MegaOhm Zentimeter aufweisen.

Die vorliegende Offenbarung beinhaltet zudem eine der vorgenannten Anordnungen mit Einfasskörper (5), wobei der stabförmige Isolierkörper (3) und der Einfasskörper (5) jeweils elektrische Isolatoren sind und bei Temperaturen von 393 Kelvin jeweils einen spezifischen elektrischen Widerstand $\rho$ von mindestens zehn MegaOhm Zentimeter aufweisen.

Die vorliegende Offenbarung beinhaltet weiterhin eine der vorgenannten Anordnungen mit Einfasskörper (5), wobei der stabförmige Isolierkörper (3) und der Einfasskörper (5) jeweils elektrische Isolatoren sind und bei Temperaturen von 473 Kelvin jeweils einen spezifischen elektrischen Widerstand $\rho$ von mindestens einem MegaOhm Zentimeter aufweisen.

Die vorliegende Offenbarung beinhaltet ausserdem eine der vorgenannten Anordnungen mit Einfasskörper (5), wobei der stabförmige Isolierkörper (3) und der Einfasskörper (5) jeweils elektrische Isolatoren sind und bei Temperaturen von 473 Kelvin jeweils einen spezifischen elektrischen Widerstand $\rho$ von mindestens

zehn MegaOhm Zentimeter aufweisen.

Die vorliegende Offenbarung beinhaltet ferner eine der vorgenannten Anordnungen mit Einfasskörper (5), wobei der stabförmige Isolierkörper (3) und der Einfasskörper (5) jeweils elektrische Isolatoren sind und bei Temperaturen von 673 Kelvin jeweils einen spezifischen elektrischen Widerstand $\rho$ von mindestens einem MegaOhm Zentimeter aufweisen.

Die vorliegende Offenbarung beinhaltet darüber hinaus eine der vorgenannten Anordnungen mit Einfasskörper (5), wobei der stabförmige Isolierkörper (3) und der Einfasskörper (5) jeweils elektrische Isolatoren sind und bei Temperaturen von 673 Kelvin jeweils einen spezifischen elektrischen Widerstand $\rho$ von mindestens zehn MegaOhm Zentimeter aufweisen.

Die vorliegende Offenbarung beinhaltet zudem eine der vorgenannten Anordnungen mit Einfasskörper (5), wobei der stabförmige Isolierkörper (3) und der Einfasskörper (5) jeweils elektrische Isolatoren sind und bei Temperaturen von 873 Kelvin jeweils einen spezifischen elektrischen Widerstand $\rho$ von mindestens einem MegaOhm Zentimeter aufweisen.

Die vorliegende Offenbarung beinhaltet weiterhin eine der vorgenannten Anordnungen mit Einfasskörper (5), wobei der stabförmige Isolierkörper (3) und der Einfasskörper (5) jeweils elektrische Isolatoren sind und bei Temperaturen von 873 Kelvin jeweils einen spezifischen elektrischen Widerstand $\rho$ von mindestens zehn MegaOhm Zentimeter aufweisen.

Die vorliegende Offenbarung lehrt ferner eine der vorgenannten Anordnungen,
wobei die Abstandshalter (5, 6a, 6b, 7a, 7b) einen ersten Ring (6a, 6b), der entlang einer ersten geschlossenen Kurve um den stabförmigen Isolierkörper (3) herum verläuft und an den stabförmigen Isolierkörper (3) grenzt und an die Ummantelung (2) grenzt, umfassen.

Die vorliegende Offenbarung beinhaltet zudem eine der vorgenannten Anordnungen, wobei die Abstandshalter (5, 6a, 6b, 7a, 7b) einen ersten Ring (6a, 6b), der entlang einer ersten geschlossenen Kurve (aussen) um den stabförmigen Isolierkörper (3) herum verläuft und den stabförmigen Isolierkörper (3) berührt und die Ummantelung (2) berührt, umfassen.

Die vorliegende Offenbarung beinhaltet ferner eine der vorgenannten Anordnungen, wobei die Abstandshalter (5, 6a, 6b, 7a, 7b) einen ersten Ring (6a, 6b), der entlang einer ersten geschlossenen Kurve (aussen) um den stabförmigen Isolierkörper (3) herum verläuft und an die Aussenoberfläche des stabförmigen Isolierkörpers (3) grenzt und an die Innenoberfläche der Ummantelung (2) grenzt, umfassen.

Die vorliegende Offenbarung beinhaltet weiterhin eine der vorgenannten Anordnungen, wobei die Abstandshalter (5, 6a, 6b, 7a, 7b) einen ersten Ring (6a, 6b), der entlang einer ersten geschlossenen Kurve (aussen) um den stabförmigen Isolierkörper (3) herum verläuft und die Aussenoberfläche des stabförmigen Isolierkörpers (3) berührt und die Innenoberfläche der Ummantelung (2) berührt, umfassen.

Die erste geschlossenen Kurve kann eine erste geschlossene Linie sein. Die erste geschlossenen Kurve kann ein erster Kreis und/oder eine erste Ellipse sein.

Die vorliegende Offenbarung lehrt weiterhin eine der vorgenannten Anordnungen mit erstem Ring (6a, 6b), wobei der erste Ring (6a, 6b) einen ersten Abstand zum ersten Ende (4a) aufweist und einen zweiten Abstand zum zweiten Ende (4b) aufweist, wobei der erste Abstand des ersten Rings (6a, 6b) von dem ersten Ende (4a) grösser als der zweite Abstand des ersten Rings (6a, 6b) von dem zweiten Ende (4b) ist.

Die vorliegende Offenbarung beinhaltet ferner eine der vorgenannten Anordnungen mit erstem Ring (6a, 6b), wobei der erste Ring (6a, 6b) einen ersten Abstand zum ersten Ende (4a) aufweist und einen zweiten Abstand zum zweiten Ende (4b) aufweist, wobei der erste Abstand des ersten Rings (6a, 6b) von dem ersten Ende (4a) länger als der zweite Abstand des ersten Rings (6a, 6b) von dem zweiten Ende (4b) ist.

Die vorliegende Offenbarung beinhaltet weiterhin eine der vorgenannten Anordnungen mit erstem Ring (6a, 6b), wobei der erste Ring (6a, 6b) einen ersten Abstand zur ersten Öffnung der Ummantelung (2) aufweist und einen zweiten Abstand zur zweiten Öffnung der Ummantelung (2) aufweist, wobei der erste Abstand des ersten Rings (6a, 6b) von der ersten Öffnung der Ummantelung (2) grösser als der zweite Abstand des ersten Rings (6a, 6b) von der zweiten Öffnung der Ummantelung (2) ist.

Die vorliegende Offenbarung beinhaltet darüber hinaus eine der vorgenannten Anordnungen mit erstem Ring (6a, 6b), wobei der erste Ring (6a, 6b) einen ersten Abstand zur ersten Öffnung der Ummantelung (2) aufweist und einen zweiten Abstand zur zweiten Öffnung der Ummantelung (2) aufweist, wobei der erste Abstand des ersten Rings (6a, 6b) von der ersten Öffnung der Ummantelung (2) länger als der zweite Abstand des ersten Rings (6a, 6b) von der zweiten Öffnung der Ummantelung (2) ist.

Die vorliegende Offenbarung lehrt weiterhin eine der vorgenannten Anordnungen mit Einfasskörper (5) und mit erstem Ring (6a, 6b), wobei der Einfasskörper (5) eine Aussparung umfasst und der erste Ring (6a, 6b) einen Abschnitt umfasst, der in der Aussparung des Einfasskörpers (5) angeordnet ist und/oder verläuft.

Die vorliegende Offenbarung beinhaltet weiterhin eine der vorgenannten Anordnungen mit Einfasskörper (5) und mit erstem Ring (6a, 6b), wobei der Einfasskörper (5) eine Rille umfasst und der erste Ring (6a, 6b) einen (zylindersymmetrischen) Abschnitt umfasst, der in der Rille des Einfasskörpers (5) angeordnet ist und/oder verläuft.

Die vorliegende Offenbarung beinhaltet zudem eine der vorgenannten Anordnungen mit Einfasskörper (5) und mit erstem Ring (6a, 6b), wobei der Einfasskörper (5) eine Furche umfasst und der erste Ring (6a, 6b) einen (zylindersymmetrischen) Abschnitt umfasst, der in der Furche des Einfasskörpers (5) angeordnet ist und/oder verläuft.

Die vorliegende Offenbarung lehrt weiterhin eine der vorgenannten Anordnungen, wobei die Abstandshalter (5, 6a, 6b, 7a, 7b) einen zweiten Ring (7a, 7b), der entlang einer zweiten geschlossenen Kurve um den stabförmigen Isolierkörper (3) herum verläuft und an den stabförmigen Isolierkörper (3) grenzt und an die Ummantelung (2) grenzt und verschieden von einem oder von dem ersten Ring (6a, 6b) ist, umfassen; und wobei der zweite Ring (7a, 7b) einen ersten Abstand zum ersten Ende (4a) aufweist und einen zweiten Abstand zum zweiten Ende (4b) aufweist, wobei der erste Abstand des zweiten Rings (7a, 7b) von dem ersten Ende (4a) kleiner als der zweite Abstand des zweiten Rings (7a, 7b) von dem zweiten Ende (4b) ist.

Die vorliegende Offenbarung beinhaltet ausserdem eine der vorgenannten Anordnungen, wobei die Abstandshalter (5, 6a, 6b, 7a, 7b) einen zweiten Ring (7a, 7b), der entlang einer zweiten geschlossenen Kurve um den stabförmigen Isolierkörper (3) herum verläuft und an den stabförmigen Isolierkörper (3) grenzt und an die Ummantelung (2) grenzt und verschieden von einem oder von dem ersten Ring (6a, 6b) ist, umfassen; und wobei der zweite Ring (7a, 7b) einen ersten Abstand zum ersten Ende (4a) aufweist und einen zweiten Abstand zum zweiten Ende (4b) aufweist, wobei der erste Abstand des zweiten Rings (7a, 7b) von dem ersten Ende (4a) geringer und/oder kürzer als der zweite Abstand des zweiten Rings (7a, 7b) von dem zweiten Ende (4b) ist.

Die vorliegende Offenbarung beinhaltet zudem eine der vorgenannten Anordnungen, wobei die Abstandshalter (5, 6a, 6b, 7a, 7b) einen zweiten Ring (7a, 7b), der entlang einer zweiten geschlossenen Kurve um den stabförmigen Isolierkörper (3) herum verläuft und den stabförmigen Isolierkörper (3) berührt und die Ummantelung (2) berührt und verschieden von einem oder von dem ersten Ring (6a, 6b) ist, umfassen; und wobei der zweite Ring (7a, 7b) einen ersten Abstand zum ersten Ende (4a) aufweist und einen zweiten Abstand zum zweiten Ende (4b) aufweist, wobei der erste Abstand des zweiten Rings (7a, 7b) von dem ersten Ende (4a) kleiner und/oder geringer und/oder kürzer als der zweite Abstand des zweiten Rings (7a, 7b) von dem zweiten Ende (4b) ist.

Die vorliegende Offenbarung beinhaltet ferner eine der vorgenannten Anordnungen, wobei die Abstandshalter (5, 6a, 6b, 7a, 7b) einen zweiten Ring (7a, 7b), der entlang einer zweiten geschlossenen Kurve um den stabförmigen Isolierkörper (3) herum verläuft und an die Aussenoberfläche des stabförmigen Isolierkörpers (3) grenzt und an die Innenoberfläche der Ummantelung (2) grenzt und verschieden von einem oder von dem ersten Ring (6a, 6b) ist, umfassen; und wobei der zweite Ring (7a, 7b) einen ersten Abstand zum ersten Ende (4a) aufweist und einen zweiten Abstand zum zweiten Ende (4b) aufweist, wobei der erste Abstand des zweiten Rings (7a, 7b) von dem ersten Ende (4a) kleiner und/oder geringer und/oder kürzer als der zweite Abstand des zweiten Rings (7a, 7b) von dem zweiten Ende (4b) ist.

Die vorliegende Offenbarung beinhaltet weiterhin eine der vorgenannten Anordnungen, wobei die Abstandshalter (5, 6a, 6b, 7a, 7b) einen zweiten Ring (7a, 7b), der entlang einer zweiten geschlossenen Kurve um den stabförmigen Isolierkörper (3) herum verläuft und die Aussenoberfläche des stabförmigen Isolierkörpers (3) berührt und die Innenoberfläche der Ummantelung (2) berührt und verschieden von einem oder von dem ersten Ring (6a, 6b) ist, umfassen; und

wobei der zweite Ring (7a, 7b) einen ersten Abstand zum ersten Ende (4a) aufweist und einen zweiten Abstand zum zweiten Ende (4b) aufweist, wobei der erste Abstand des zweiten Rings (7a, 7b) von dem ersten Ende (4a) kleiner und/oder geringer und/oder kürzer als der zweite Abstand des zweiten Rings (7a, 7b) von dem zweiten Ende (4b) ist.

Die zweite geschlossene Kurve ist verschieden von der ersten geschlossenen Kurve. Die zweite geschlossenen Kurve kann eine zweite geschlossene Linie sein. Die zweite geschlossenen Kurve kann ein zweiter Kreis und/oder eine zweite Ellipse sein.

Die vorliegende Offenbarung lehrt weiterhin eine Verbrennungsvorrichtung umfassend einen Feuerraum und eine Struktur ausgewählt aus

- einem Abgaskamin,
- einem Rauchgaskamin,
- einem Schornstein;

    wobei die Struktur mit dem Feuerraum in Fluidverbindung ist;
    wobei die Verbrennungsvorrichtung eine der vorgenannten Anordnungen umfasst; und
    wobei mindestens ein Abschnitt (10b) der Anordnung innerhalb der Struktur angeordnet ist.

Die vorliegende Offenbarung lehrt ferner eine der vorgenannten Verbrennungsvorrichtungen, wobei die Struktur eine Aussenwand (9) umfasst und die Ummantelung (2) der Anordnung so durch die Aussenwand (9) geführt ist, dass der mindestens eine Abschnitt (10b) der Anordnung in die Struktur hineinragt.

Die vorliegende Offenbarung beinhaltet weiterhin eine der vorgenannten Verbrennungsvorrichtungen mit Aussenwand (9), wobei die Anordnung eine Durchführung durch die Aussenwand (9) bildet und/oder ist.

Die vorliegende Offenbarung beinhaltet ferner eine der vorgenannten Verbrennungsvorrichtungen mit Aussenwand (9), wobei die Aussenwand (9) und die Ummantelung (2) der Anordnung jeweils einen Temperaturausdehnungskoeffizienten $\alpha$ zwischen $10.10^{-6}$/Kelvin und $20.10^{-6}$/Kelvin bei 293 Kelvin aufweisen.

Die vorliegende Offenbarung beinhaltet ausserdem eine der vorgenannten Verbrennungsvorrichtungen mit Aussenwand (9), wobei die Aussenwand (9) und die Ummantelung (2) der Anordnung jeweils einen Temperaturausdehnungskoeffizienten $\alpha$ zwischen $10.10^{-6}$/Kelvin und $20.10^{-6}$/Kelvin in einem Bereich zwischen 273 Kelvin und 873 Kelvin, insbesondere in einem Bereich zwischen 273 Kelvin und 673 Kelvin, besonders bevorzugt in einem Temperaturbereich zwischen 273 Kelvin und 473 Kelvin, aufweisen.

Das Genannte bezieht sich auf einzelne Ausführungsformen der Offenbarung. Verschiedene Änderungen an den Ausführungsformen können vorgenommen werden, ohne von der zu Grunde liegenden Idee abzuweichen und ohne den Rahmen dieser Offenbarung zu verlassen. Der Gegenstand der vorliegenden Offenbarung ist definiert über deren Ansprüche. Es können verschiedenste Änderungen vorgenommen werden, ohne den Schutzbereich der folgenden Ansprüche zu verlassen.

Bezugszeichen

    1a, 1b, 1c: Zuleitungen
    2: Ummantelung
    3: stabförmiger Isolierkörper
    4a, 4b: Enden
    5: Einfasskörper
    6a, 6b: Ring
    7a, 7b: Ring
    8a, 8b: Ring
    9 Wand
    10a, 10b: Abschnitte

## Patentansprüche

1. Anordnung umfassend mindestens eine erste elektrisch leitfähige Zuleitung (1a, 1b, 1c), eine Ummantelung (2) und einen stabförmigen Isolierkörper (3);

    wobei die Ummantelung (2) ein erstes Ende (4a) und ein zweites Ende (4b) aufweist und das erste Ende (4a) verschieden von dem zweiten Ende (4b) ist und das erste Ende (4a) dem zweiten Ende (4b) gegenüberliegt und die Ummantelung (2) eine erste Öffnung an dem ersten Ende (4a) und eine zweite Öffnung an dem zweiten Ende (4b) aufweist;
    wobei die mindestens eine erste Zuleitung (1a, 1b, 1c) von dem ersten Ende (4a) zum zweiten Ende (4b) durch die Anordnung hindurch verläuft und eine Achse definiert;
    wobei der stabförmige Isolierkörper (3) mindestens einen Durchgang durch den stabförmigen Isolierkörper (3) umfasst und ein erster Abschnitt der mindestens einen Zuleitung (1a, 1b, 1c) in dem mindestens einen Durchgang durch den stabförmigen Isolierkörper (3) verläuft;

wobei die mindestens eine erste Zuleitung (1a, 1b, 1c) zwischen der ersten Öffnung und der zweiten Öffnung eine erste Länge aufweist; wobei der erste Abschnitt der mindestens einen Zuleitung (1a, 1b, 1c) in dem mindestens einen Durchgang durch den stabförmigen Isolierkörper (3) eine zweite Länge aufweist und die zweite Länge kleiner als die erste Länge ist; wobei der stabförmige Isolierkörper (3) radial nach aussen von der Achse aus eine Aussenoberfläche umfasst und die Ummantelung (2) radial nach aussen von der Achse aus eine Innenoberfläche umfasst, wobei die Aussenoberfläche des stabförmigen Isolierkörpers (3) und die Innenoberfläche der Ummantelung (2) jeweils parallel zur Achse verlaufen, sodass die Aussenoberfläche des stabförmigen Isolierkörpers (3) und die Innenoberfläche der Ummantelung (2) einander gegenüberliegen; und wobei die Anordnung einen Spalt mit einer Dicke umfasst, der zwischen der Aussenoberfläche des stabförmigen Isolierkörpers (3) und der Innenoberfläche der Ummantelung (2) angeordnet ist, und Abstandshalter (5, 6a, 6b, 7a, 7b) zur Beabstandung des stabförmigen Isolierkörpers (3) von der Ummantelung (2) umfasst, wobei die Abstandshalter (5, 6a, 6b, 7a, 7b) die Dicke des Spaltes definieren.

2. Die Anordnung gemäss Anspruch 1,
   wobei die Abstandshalter (5, 6a, 6b, 7a, 7b) zur Beabstandung der Aussenoberfläche des stabförmigen Isolierkörpers (3) von der Innenoberfläche der Ummantelung (2) ausgebildet sind und die Dicke des Spaltes definieren.

3. Die Anordnung gemäss einem der Ansprüche 1 bis 2,
   wobei die mindestens eine erste Zuleitung (1a, 1b, 1c) von dem ersten Ende (4a) zum zweiten Ende (4b) geradelinig durch die Anordnung hindurch verläuft und die Achse definiert.

4. Die Anordnung gemäss einem der Ansprüche 1 bis 3,

   wobei die Abstandshalter (5, 6a, 6b, 7a, 7b) einen Einfasskörper (5) umfassen; und wobei der Einfasskörper (5) mindestens einen ersten Abschnitt, der senkrecht zur Achse verläuft und an den stabförmigen Isolierkörper (3) grenzt, umfasst und mindestens einen zweiten Abschnitt, der parallel zur Achse verläuft und an die Ummantelung (2) grenzt, umfasst.

5. Die Anordnung gemäss Anspruch 4,
   wobei der Einfasskörper (5) mindestens einen Durchgang aufweist und ein zweiter Abschnitt der mindestens einen Zuleitung (1a, 1b, 1c) in dem mindestens einen Durchgang durch den Einfasskörper (5) verläuft.

6. Die Anordnung gemäss einem der Ansprüche 4 bis 5,
   wobei der Einfasskörper (5) auf den stabförmigen Isolierkörper (3) von der Achse aus in axialer Richtung aufsteckbar ist.

7. Die Anordnung gemäss einem der Ansprüche 4 bis 6,
   wobei der Einfasskörper (5) in die Ummantelung (2) von der Achse aus in axialer Richtung einsteckbar ist.

8. Die Anordnung gemäss einem der Ansprüche 4 bis 7,
   wobei der Einfasskörper (5) einen ersten Abstand zum ersten Ende (4a) aufweist und einen zweiten Abstand zum zweiten Ende (4b) aufweist, wobei der erste Abstand des Einfasskörpers (5) von dem ersten Ende (4a) grösser als der zweite Abstand des Einfasskörpers (5) von dem zweiten Ende (4b) ist.

9. Die Anordnung gemäss einem der Ansprüche 4 bis 8,
   wobei der stabförmige Isolierkörper (3) und der Einfasskörper (5) jeweils elektrische Isolatoren sind und bei Temperaturen von 393 Kelvin jeweils einen spezifischen elektrischen Widerstand $\rho$ von mindestens einem MegaOhm Zentimeter aufweisen.

10. Die Anordnung gemäss einem der Ansprüche 1 bis 9,
    wobei die Abstandshalter (5, 6a, 6b, 7a, 7b) einen ersten Ring (6a, 6b), der entlang einer ersten geschlossenen Kurve um den stabförmigen Isolierkörper (3) herum verläuft und an den stabförmigen Isolierkörper (3) grenzt und an die Ummantelung (2) grenzt, umfassen.

11. Die Anordnung gemäss Anspruch 10,
    wobei der erste Ring (6a, 6b) einen ersten Abstand zum ersten Ende (4a) aufweist und einen zweiten Abstand zum zweiten Ende (4b) aufweist, wobei der erste Abstand des ersten Rings (6a, 6b) von dem ersten Ende (4a) grösser als der zweite Abstand des ersten Rings (6a, 6b) von dem zweiten Ende (4b) ist.

12. Die Anordnung gemäss Anspruch 10 und gemäss einem der Ansprüche 4 bis 9,
    wobei der Einfasskörper (5) eine Aussparung umfasst und der erste Ring (6a, 6b) einen Abschnitt umfasst, der in der Aussparung des Einfasskörpers (5) angeordnet ist und/oder verläuft.

**13.** Die Anordnung gemäss einem der Ansprüche 1 bis 12,

wobei die Abstandshalter (5, 6a, 6b, 7a, 7b) einen zweiten Ring (7a, 7b), der entlang einer zweiten geschlossenen Kurve um den stabförmigen Isolierkörper (3) herum verläuft und an den stabförmigen Isolierkörper (3) grenzt und an die Ummantelung (2) grenzt und verschieden von einem oder von dem ersten Ring (6a, 6b) ist, umfassen; und

wobei der zweite Ring (7a, 7b) einen ersten Abstand zum ersten Ende (4a) aufweist und einen zweiten Abstand zum zweiten Ende (4b) aufweist, wobei der erste Abstand des zweiten Rings (7a, 7b) von dem ersten Ende (4a) kleiner als der zweite Abstand des zweiten Rings (7a, 7b) von dem zweiten Ende (4b) ist.

**14.** Verbrennungsvorrichtung umfassend einen Feuerraum und eine Struktur ausgewählt aus

- einem Abgaskamin,
- einem Rauchgaskamin,
- einem Schornstein;

wobei die Struktur mit dem Feuerraum in Fluidverbindung ist;

wobei die Verbrennungsvorrichtung eine Anordnung gemäss einem der Ansprüche 1 bis 13 umfasst; und

wobei mindestens ein Abschnitt (10b) der Anordnung innerhalb der Struktur angeordnet ist.

**15.** Die Verbrennungsvorrichtung gemäss Anspruch 14, wobei die Struktur eine Aussenwand (9) umfasst und die Ummantelung (2) der Anordnung so durch die Aussenwand (9) geführt ist, dass der mindestens eine Abschnitt (10b) der Anordnung in die Struktur hineinragt.

**Geänderte Patentansprüche gemäss Regel 137(2) EPÜ.**

**1.** Anordnung umfassend mindestens eine erste elektrisch leitfähige Zuleitung (1a, 1b, 1c), eine Ummantelung (2) und einen stabförmigen Isolierkörper (3), der aus einem elektrisch isolierenden und temperaturbeständigen Material gefertigt ist;

wobei die Ummantelung (2) ein erstes Ende (4a) und ein zweites Ende (4b) aufweist und das erste Ende (4a) verschieden von dem zweiten Ende (4b) ist und das erste Ende (4a) dem zweiten Ende (4b) gegenüberliegt und die Ummantelung (2) eine erste Öffnung an dem ersten Ende (4a) und eine zweite Öffnung an dem zweiten Ende (4b) aufweist;

wobei die mindestens eine erste Zuleitung (1a, 1b, 1c) von dem ersten Ende (4a) zum zweiten Ende (4b) durch die Anordnung hindurch verläuft und eine Achse definiert;

wobei der stabförmige Isolierkörper (3) mindestens einen Durchgang durch den stabförmigen Isolierkörper (3) umfasst und ein erster Abschnitt der mindestens einen Zuleitung (1a, 1b, 1c) in dem mindestens einen Durchgang durch den stabförmigen Isolierkörper (3) verläuft;

wobei die mindestens eine erste Zuleitung (1a, 1b, 1c) zwischen der ersten Öffnung und der zweiten Öffnung eine erste Länge aufweist;

wobei der erste Abschnitt der mindestens einen Zuleitung (1a, 1b, 1c) in dem mindestens einen Durchgang durch den stabförmigen Isolierkörper (3) eine zweite Länge aufweist;

wobei der stabförmige Isolierkörper (3) radial nach aussen von der Achse aus eine Aussenoberfläche umfasst und die Ummantelung (2) radial nach aussen von der Achse aus eine Innenoberfläche umfasst, wobei die Aussenoberfläche des stabförmigen Isolierkörpers (3) und die Innenoberfläche der Ummantelung (2) jeweils parallel zur Achse verlaufen, sodass die Aussenoberfläche des stabförmigen Isolierkörpers (3) und die Innenoberfläche der Ummantelung (2) einander gegenüberliegen; und

wobei die Anordnung einen Spalt mit einer Dicke umfasst, der zwischen der Aussenoberfläche des stabförmigen Isolierkörpers (3) und der Innenoberfläche der Ummantelung (2) angeordnet ist, und Abstandshalter (5, 6a, 6b, 7a, 7b) zur Beabstandung des stabförmigen Isolierkörpers (3) von der Ummantelung (2) umfasst, wobei die Abstandshalter (5, 6a, 6b, 7a, 7b) die Dicke des Spaltes definieren,

**dadurch gekennzeichnet, dass**

die zweite Länge kleiner als die erste Länge ist; und

die Abstandshalter (5, 6a, 6b, 7a, 7b) einen ersten Ring (6a, 6b), der entlang einer ersten geschlossenen Kurve um den stabförmigen Isolierkörper (3) herum verläuft und an den stabförmigen Isolierkörper (3) grenzt und an die Ummantelung (2) grenzt, umfassen.

**2.** Die Anordnung gemäss Anspruch 1, wobei die Abstandshalter (5, 6a, 6b, 7a, 7b) zur Beabstandung der Aussenoberfläche des stabförmigen Isolierkörpers (3) von der Innenoberfläche der Ummantelung (2) ausgebildet sind und die Dicke des Spaltes definieren.

**3.** Die Anordnung gemäss einem der Ansprüche 1 bis 2, wobei die mindestens eine erste Zuleitung (1a, 1b, 1c) von dem ersten Ende (4a) zum zweiten Ende (4b) geradelinig durch die Anordnung hindurch verläuft und die Achse definiert.

4. Die Anordnung gemäss einem der Ansprüche 1 bis 3,
   wobei die Abstandshalter (5, 6a, 6b, 7a, 7b) einen Einfasskörper (5) umfassen; und wobei der Einfasskörper (5) mindestens einen ersten Abschnitt, der senkrecht zur Achse verläuft und an den stabförmigen Isolierkörper (3) grenzt, umfasst und mindestens einen zweiten Abschnitt, der parallel zur Achse verläuft und an die Ummantelung (2) grenzt, umfasst.

5. Die Anordnung gemäss Anspruch 4,
   wobei der Einfasskörper (5) mindestens einen Durchgang aufweist und ein zweiter Abschnitt der mindestens einen Zuleitung (1a, 1b, 1c) in dem mindestens einen Durchgang durch den Einfasskörper (5) verläuft.

6. Die Anordnung gemäss einem der Ansprüche 4 bis 5,
   wobei der Einfasskörper (5) auf den stabförmigen Isolierkörper (3) von der Achse aus in axialer Richtung aufsteckbar ist.

7. Die Anordnung gemäss einem der Ansprüche 4 bis 6,
   wobei der Einfasskörper (5) in die Ummantelung (2) von der Achse aus in axialer Richtung einsteckbar ist.

8. Die Anordnung gemäss einem der Ansprüche 4 bis 7,
   wobei der Einfasskörper (5) einen ersten Abstand zum ersten Ende (4a) aufweist und einen zweiten Abstand zum zweiten Ende (4b) aufweist, wobei der erste Abstand des Einfasskörpers (5) von dem ersten Ende (4a) grösser als der zweite Abstand des Einfasskörpers (5) von dem zweiten Ende (4b) ist.

9. Die Anordnung gemäss einem der Ansprüche 4 bis 8,
   wobei der stabförmige Isolierkörper (3) und der Einfasskörper (5) jeweils elektrische Isolatoren sind und bei Temperaturen von 393 Kelvin jeweils einen spezifischen elektrischen Widerstand $\rho$ von mindestens einem MegaOhm·Zentimeter aufweisen.

10. Die Anordnung gemäss Anspruch 1,
    wobei der erste Ring (6a, 6b) einen ersten Abstand zum ersten Ende (4a) aufweist und einen zweiten Abstand zum zweiten Ende (4b) aufweist, wobei der erste Abstand des ersten Rings (6a, 6b) von dem ersten Ende (4a) grösser als der zweite Abstand des ersten Rings (6a, 6b) von dem zweiten Ende (4b) ist.

11. Die Anordnung gemäss einem der Ansprüche 4 bis 9,
    wobei der Einfasskörper (5) eine Aussparung umfasst und der erste Ring (6a, 6b) einen Abschnitt umfasst, der in der Aussparung des Einfasskörpers (5) angeordnet ist und/oder verläuft.

12. Die Anordnung gemäss einem der Ansprüche 1 bis 11,
    wobei die Abstandshalter (5, 6a, 6b, 7a, 7b) einen zweiten Ring (7a, 7b), der entlang einer zweiten geschlossenen Kurve um den stabförmigen Isolierkörper (3) herum verläuft und an den stabförmigen Isolierkörper (3) grenzt und an die Ummantelung (2) grenzt und verschieden von einem oder von dem ersten Ring (6a, 6b) ist, umfassen; und
    wobei der zweite Ring (7a, 7b) einen ersten Abstand zum ersten Ende (4a) aufweist und einen zweiten Abstand zum zweiten Ende (4b) aufweist, wobei der erste Abstand des zweiten Rings (7a, 7b) von dem ersten Ende (4a) kleiner als der zweite Abstand des zweiten Rings (7a, 7b) von dem zweiten Ende (4b) ist.

13. Verbrennungsvorrichtung umfassend einen Feuerraum und eine Struktur ausgewählt aus

    - einem Abgaskamin,
    - einem Rauchgaskamin,
    - einem Schornstein;

      wobei die Struktur mit dem Feuerraum in Fluidverbindung ist;
      wobei die Verbrennungsvorrichtung eine Anordnung gemäss einem der Ansprüche 1 bis 12 umfasst; und
      wobei mindestens ein Abschnitt (10b) der Anordnung innerhalb der Struktur angeordnet ist.

14. Die Verbrennungsvorrichtung gemäss Anspruch 13, wobei die Struktur eine Aussenwand (9) umfasst und die Ummantelung (2) der Anordnung so durch die Aussenwand (9) geführt ist, dass der mindestens eine Abschnitt (10b) der Anordnung in die Struktur hineinragt.

FIG 1

FIG 2

**Europäisches Patentamt**
**European Patent Office**
**Office européen des brevets**

# EUROPÄISCHER RECHERCHENBERICHT

Nummer der Anmeldung

EP 23 21 1581

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | CH 592 950 A5 (ASEA AB) 15. November 1977 (1977-11-15) | 1,3 | INV. G01N27/406 |
| A | * Fig. 2 und korrespondierende Textpassagen * | 2,4-15 | ADD. |
| | ----- | | G01N27/407 |
| Y | WO 2017/016820 A1 (R STAHL SCHALTGERÄTE GMBH [DE]) 2. Februar 2017 (2017-02-02) * Fig. 8,11 und korrespondierende Textpassagen * | 1,3 | |
| | ----- | | |
| A | EP 0 789 437 A2 (ASEA BROWN BOVERI [CH]) 13. August 1997 (1997-08-13) * Fig. 1,2 und korrespondierende Textpassagen * | 1-15 | |
| | ----- | | |

RECHERCHIERTE SACHGEBIETE (IPC)

G01N
H01B
H01G

Der vorliegende Recherchenbericht wurde für alle Patentansprüche erstellt

| Recherchenort | Abschlußdatum der Recherche | Prüfer |
|---|---|---|
| München | 22. April 2024 | Klein, Marc-Oliver |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 21 1581

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-04-2024

| Im Recherchenbericht angeführtes Patentdokument | | Datum der Veröffentlichung | Mitglied(er) der Patentfamilie | | | Datum der Veröffentlichung |
|---|---|---|---|---|---|---|
| CH 592950 | A5 | 15-11-1977 | CH | 592950 | A5 | 15-11-1977 |
| | | | DE | 2549018 | A1 | 26-05-1976 |
| | | | GB | 1516718 | A | 05-07-1978 |
| | | | SE | 386762 | B | 16-08-1976 |
| | | | US | 3973077 | A | 03-08-1976 |
| WO 2017016820 | A1 | 02-02-2017 | CN | 108028518 | A | 11-05-2018 |
| | | | DE | 102015112284 | A1 | 02-02-2017 |
| | | | EP | 3329567 | A1 | 06-06-2018 |
| | | | US | 2018226781 | A1 | 09-08-2018 |
| | | | WO | 2017016820 | A1 | 02-02-2017 |
| EP 0789437 | A2 | 13-08-1997 | BR | 9700898 | A | 12-01-1999 |
| | | | CA | 2195138 | A1 | 09-08-1997 |
| | | | DE | 19604481 | A1 | 14-08-1997 |
| | | | EP | 0789437 | A2 | 13-08-1997 |
| | | | JP | H09219919 | A | 19-08-1997 |
| | | | PL | 318152 | A1 | 18-08-1997 |
| | | | US | 6002084 | A | 14-12-1999 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 2685875 Y
- WO 2022064271 A1
- DE 102012211039 A1
- US 6015533 A
- US 2010050738 A1
- EP 4236640 A1
- CN 115791931 A